# EUROPÄISCHE PATENTANMELDUNG

(11) **EP 1 738 774 A1**
(43) Veröffentlichungstag der Anmeldung: **03.01.2007**
(21) Anmeldenummer: 05014059.9
(22) Anmeldetag: 29.06.2005
(51) Int. Cl.: A61K 49/18

(54) **Magnetische Eisenoxidpartikel enthaltende Zusammensetzungen und deren Verwendung in bildgebenden Verfahren**

(71) Anmelder: Schering AG, 13342 Berlin (DE); Philips Intellectual Property & Standards GmbH, 20099 Hamburg (DE); Koninklijke Philips Electronics N.V., 5621 BA Eindhoven (NL)
(72) Erfinder: Briel, Andreas, 10245 Berlin (DE); Gleich, Bernhard, 22335 Hamburg (DE); Weizenecker, Jürgen, 22415 Hamburg (DE); Rohrer, Martin, 12203 Berlin (DE); Weinmann Hanns-Joachim, 14129 Berlin (DE); Pietsch, Hubertus, 13127 Berlin (DE); Lawaczeck, Rüdiger, 13503 Berlin (DE); Rothe, Matthias, 14167 Berlin (DE); Thomsen, Jens, 10781 Berlin (DE)
(74) Vertreter: Engelhard, Markus

(57) **Zusammenfassung**

Die vorliegende Erfindung betrifft Komplexe, die in einer pharmazeutisch annehmbaren Umhüllung magnetische Eisenoxidpartikel mit einem Durchmesser von 20 nm bis 1 µm bei einem Gesamtpartikeldurchmesser-Kerndurchmesserverhältnis, das kleiner als 6 ist, enthalten, sowie die Verwendung dieser Komplexe bei der Bildgebung durch Detektion magnetischer Partikel, die auch als "magnetic particle imaging" (MPI) bezeichnet wird. Insbesondere ist hierbei die Verwendung dieser Zusammensetzungen bei der Untersuchung des Magen-Darmtrakts, des Blutgefäßsystems des Herzens und cranialer Komponenten, bei der Diagnose von Arteriosklerose, Infarkten und Tumoren und Metastasen, beispielsweise des Lymphsystems bevorzugt.

## Beschreibung

### Einleitung

Die vorliegende Erfindung betrifft Komplexe, die in einer pharmazeutisch annehmbaren Umhüllung magnetische Eisenoxidpartikel mit einem Durchmesser von 20 nm bis 1 µm bei einem Gesamtpartikeldurchmesser-Kerndurchmesserverhältnis das kleiner als 6 ist, enthalten, sowie die Verwendung dieser Komplexe bei der Bildgebung durch Detektion magnetischer Partikel, die auch als "magnetic particle imaging" (MPI) bezeichnet wird. Insbesondere ist hierbei die Verwendung dieser Zusammensetzungen bei der Untersuchung des Magen-Darmtrakts, des Blutgefäßsystems des Herzens und cranialer Komponenten, bei der Diagnose von Arteriosklerose, Infarkten und Tumoren und Metastasen, beispielsweise des Lymphsystems bevorzugt.

### Stand der Technik

Kontrastmitteln haben in bildgebenden Verfahren vor allem im Bereich der medizinischen Diagnostik zu einer deutlichen Verbesserung des Kontrasts herkömmlicher bildgebender Verfahren wie der Röntgendiagnostik und der Bildgebung durch magnetische Resonanz, auch als "magnetic resonance imaging" (MRI) bekannt, geführt. Kontrastmittel, die beim MRI eingesetzt werden können anhand ihres Wirkungsmechanismus (positive Verstärkung oder Beeinflussung der longitudinalen Relaxation bzw. negative Verstärkung oder Beeinflussung der transversalen Relaxation) unterschieden werden. Dieser Effekt wird als T₁- und T₂-Relaxivität in der Einheit mM⁻¹ * s⁻¹ ausgedrückt. R₁ und R₂ sind als die Anstiegssteilheit der Kurven 1/T₁ bzw. 1/T₂ gegen die Konzentration des Kontrastmittels definiert. Das Verhältnis R₂/R₁ bestimmt, ob ein Kontrastmittel überwiegend T₁ (R₁ signifikant höher als R₂) oder T₂-verkürzenden Effekt hat (Krombach et al. (2002) Röfo 174: 819-829). Positive Kontrastmittel (auch sog. Relaxitivitäts oder T₁-verstärkende) Kontrastmittel verstärken die Signalintensität von perfundierten Arealen. Negative (auch sogenannte Suszeptibilitäts- oder T₂-verstärkende) Kontrastmittel erniedrigen die Signalintensität von einem perfundierten Territorium bei T₂-gewichteten Sequenzen.

Geeignete MRI-Kontrastmittel sind im Stand der Technik beschrieben worden. Beispielsweise werden in der EP 0 525 199 pharmazeutische Zubereitungen mit magnetischen Eisenoxidpartikeln, die mit Polysacchariden komplexiert sind, sowie deren Verwendung als Kontrastmittel im MRI beschrieben. Die superparamagnetischen Eisenoxidkerne weisen in einer bevorzugten Ausführungsform eine Größe von 2 nm bis 30 nm auf. Die Größe der Komplexe (Kern plus Polysaccharid-Hülle) in geeigneten Zubereitungen ist mit 10 nm bis 500 nm angegeben. Eine konkrete Offenbarung erstreckt sich jedoch lediglich auf Komplexe mit Eisenoxidkernen mit einem Durchmesser von 10,1 nm. In der EP 0 543 020 werden ganz ähnliche Partikel offenbart. Allerdings sind hier die superparamagnetischen Eisenoxidpartikel mit Carboxypolysacchariden komplexiert. Des weiteren wird die Verwendung dieser Komplexe als Kontrastmittel im MRI beschrieben. Die Verwendung von Carboxydextran als Hüllenmaterial verbessert die pharmakologischen Eigenschaften der Zubereitung. Die Größe des Eisenoxidkerns beträgt in einer bevorzugten Ausführungsform 20 nm bis 30 nm. Die konkret offenbarten Komplexe enthalten jedoch nur Eisenoxidkerne mit einem Durchmesser von maximal 8,8 nm. In der US 5,492,814 werden monokristalline Eisenoxidpartikel beschrieben sowie deren Verwendung zur Untersuchung biologischen Gewebes mittels MRI. Als bevorzugte Größe der Eisenoxidkerne werden Bereiche von 1 bis 10 nm angegeben in den Beispielen konkret offenbart werden jedoch lediglich Partikel mit Eisenkernen mit einem Durchmesser von 2.9 +/- 1.3 nm. Zur Verbesserung der Eignung als Kontrastmittel bei der MRI handelt es sich bei den offenbarten Partikeln vorzugsweise um monokristalline Partikel, d.h. die Kristallstruktur des gesamten Teilchen ist homogen und störungsfrei - ein Einkristall also.

Kürzlich wurde ein neues Verfahren zur Bildgebung im medizinischen Bereich beschrieben. Dabei wird die Änderung der Magnetisierung von Partikeln in einem sich bewegenden Magnetfeld gemessen. Diese Änderung dient der Ermittlung der räumlichen Verteilung der magnetischer Partikel in einem Untersuchungsbereich (siehe bspw. die DE 101 51 778 A1 und die DE 102 38 853 A1). Diese neue Technik wurde als Magnetic Particle Imaging (MPI) bezeichnet. Diese und weitere Anmeldungen desselben Anmelders erwähnen eine Reihe von Eigenschaften, die Partikel aufweisen müssen, die in dem MPI-Verfahren verwendet werden können. Beispielsweise können die Partikel ferro- und ferrimagnetische Partikel sein, und ähneln somit den vom MRI-Verfahren bekannten Partikeln. Allerdings kommt es für die Verwendbarkeit von Partikeln beim MPI nicht auf die Beeinflussung der T₁ bzw. T₂-Relaxivität an. Wegen der grundsätzlich anderen physikalischen Phänomene, die zur Bildgebung beim MRI- bzw. MPI-Verfahren verwendet werden, kann aus der Eignung eines im Stand der Technik beschriebenen Partikels als Kontrastmittel für MRI kein Schluß auf die Eignung des Partikels für MPI gezogen werden. Des weiteren wird gelehrt, daß die Partikel so klein sein sollen, daß sich in ihnen nur eine einzige magnetische Domäne (die Monodomäne) ausbilden kann bzw. keine Weiß'schen Bereiche entstehen können. Es wird vermutet, daß geeignete Monodomänen-Partikeln je nach Material eine ideale Größe im Bereich von 20 nm bis ca. 800 nm aufweisen sollten. Magnetit (Fe₃O₄) wird als ein geeignetes Material für Monodomänen-Partikel genannt.

MPI ist eine vielversprechende neue Methode, die vor allem deshalb für diagnostische Anwendungen interessant ist, da der benötigte apparative Aufwand im Vergleich zur MRI deutlich geringer ist. Anders als beim MRI sind nämlich beim MPI keine großen homogenen Magnetfelder und damit auch nicht die gewaltigen supraleitenden Magnete erforderlich, die die MRI-Diagnostik so kostspielig machen und deren breite Anwendung erschweren. Um eine breite Verwendung dieser neuen Technologie zu ermöglichen ist es jedoch erforderlich magnetische Partikel zu entwickeln, die eine hohe örtliche Auflösung, eine gefahrlose Administration und geringe magnetische Feldstärken bei der Messung ermöglichen. Es besteht somit ein Bedarf Partikel zur Verfügung zu stellen, die für die MPI-Diagnostik geeignet sind.

### Beschreibung der Erfindung

Bevor die vorliegende Erfindung im größeren Detail unten beschrieben wird, sollte erkannt werden, daß diese Erfindung nicht auf die bestimmten hierin beschriebenen Methoden, Protokolle und Reagenzien beschränkt ist, da diese variiert werden können. Die hierin verwendete Terminologie wurde nur zum Zwecke des Beschreibens der besonderen bevorzugten Ausführungsformen verwendet und es ist nicht beabsichtigt, daß diese den Umfang der Erfindung beschränkt, der lediglich durch die angefügten Ansprüche beschränkt wird. Wenn nicht anders definiert, haben die hierin verwendeten technischen und wissenschaftlichen Begriffe die Bedeutung, die ihnen der Fachmann zumißt. Vorzugsweise werden die Begriffe hierin mit der in "A multilingual glossary of biotechnological terms: (IUPAC Recommendations)", Leuenberger, H.G.W, Nagel, B. and Klbl, H. eds. (1995), Helvetica Chimica Acta, CH-4010 Basel, Switzerland) definierten Bedeutung verwendet.

In der Beschreibung werden eine Reihe von Dokumenten zitiert. Jedes der hierin zitierten Dokumente (umfassend alle Patente, Patentanmeldungen, wissenschaftliche Publikationen, Bedienungsanleitungen, Herstellerempfehlungen etc.) wird durch Verweis im vollen Umfang hierin aufgenommen. Die Erwähnung eines dieser Dokumente soll jedoch in keinem Fall als ein Eingeständnis verstanden werden, daß der vorliegenden Erfindung nicht das Recht zukommt auf Grund eines früheren Erfindungsdatums dieser Veröffentlichung im Zeitrang vorzugehen.

Die Erfinder haben nun überraschenderweise gefunden, daß Partikel, die einen magnetischen Eisenoxidkern umfassen, der eine bestimmte Größe und ein bestimmtes Verhältnis zwischen Durchmesser des Eisenoxidkerns und des Gesamtpartikels aufweist eine besondere Eignung zur Bildgebung im MPI zeigen. Ein erster Gegenstand der vorliegenden Erfindung ist daher ein Partikel, der in einer pharmazeutisch annehmbaren Hülle einen magnetischen Eisenoxidkern mit einem Durchmesser von 20 nm bis 1 µm bei einem Gesamtpartikeldurchmesser-Kemdurchmesserverhältnis das kleiner als 6 ist, enthält.

Es ist bekannt, daß die Verabreichung reiner Eisenoxidkerne beim Patienten zu schweren Nebenwirkungen führt. Beschrieben wurden rapider Abfall des Blutdrucks sowie eine Blutplättchenaggregation. Um diese im Einzelfall lebensbedrohenden Nebenwirkungen zu vermeiden, ist der Eisenoxidkern des erfindungsgemäßen Partikels mit einer pharmazeutisch annehmbaren Hülle umgeben. Eine "pharmazeutisch annehmbare Hülle" im Sinne der vorliegenden Erfindung, ist eine den Eisenoxidkern im wesentlichen vollständig einschließende Schicht einer Substanz oder eine Substanzmischung, die den Eisenoxidkern so abschirmt, daß bei Verabreichung an einen Patienten die bekannten lebensbedrohenden Nebenwirkungen nicht auftreten. Hierbei ist es bevorzugt, daß die Substanz oder Substanzmischung bioabbaubar ist, d.h. in kleinere vom Körper verwertbare Einheiten aufgespaltet werden kann und/oder über die Niere ausgeschieden werden kann. Die Partikel werden dem Patienten vorzugsweise in einer wäßrigen kolloidalen Lösung bzw. Dispersion verabreicht. Es ist daher wünschenswert, daß die Substanz oder des Substanzgemischs hydrophil ist und die Präzipitation der Partikel verhindert bzw. die kolloidale Lösung stabilisiert. Im Stand der Technik ist eine Vielzahl solcher Substanzen beschrieben (siehe z.B. US 5,492,814).

In einer bevorzugten Ausführungsform ist die pharmazeutisch annehmbare Hülle ein synthetisches Polymer oder Copolymer, eine Stärke oder ein Derivat davon, ein Dextran oder ein Derivat davon, ein Cyclodextran oder ein Derivat davon, eine Fettsäure, ein Polysaccharid, ein Lecithin oder ein Mono-, Di- oder Triglycerid oder ein Derivat davon. Umfaßt sind auch Gemische der vorgenannten bevorzugten Stoffe.

Aus diesen breiten Substanzklassen sind die folgenden Substanzen und Gemische davon besonders bevorzugt:
(i) für Polymere oder Copolymere die Polyoxyethylensorbitanester, Polyoxyethylen und Derivate davon, Polyoxypropylen und Derivate davon, nichtionische Oberflächen-aktive Substanzen, Polyoxylstearate (35-80), Polyvinylalkoholen, polymerisierte Sucrose, Polyhydroxyalkylmethacrylamiden, Milchsäure und Glycolsäure-Copolymere, Polyorthoester, Polyalkylcyanoacrylates, Polyethylenglycole, Polypropylenglycole, Polyglycerole, polyhydroxylierte Polyvinylmatrices, Polyhydroxyethylaspartamide, Polyaminosäuren, Styrol- und Maleinsäure-Copolymere, Polycaprolactone, Carboxypolysaccharide, und Polyanhydride;
(ii) für Stärkederivate die Stärke-2-hydroymethylether und Hydroxyethylstärke;
(iii) für Dextrane oder Derivat davon die galactosylierten Dextrane, lactosylierte Dextrane, aminierte Dextrane, Dextrane mit SH-Gruppen, Dextrane mit Carboxylgruppen, Dextrane mit Aldehydgruppen, biotinylierte Dextrane;
(iv) für Cyclodextrine die beta-Cyclodextrine und die Hydroxypropylcyclodextrine;
(v) für Fettsäuren die Natriumlaurylsulfate, Natriumstearate, Stearinsäuren, Sorbitanmonolaurate, Sorbitanmonooleate, Sorbitanmonopalmitate und Sorbitanmonostearate.

Auf Grund seiner besonders guten Verträglichkeit ist die Verwendung von Dextranen und Polyethylenglykol (PEG) und Derivaten davon und insbesondere von Carboxydextranen, niedermolekularen PEGs (vorzugsweise 500 bis 2000 g/mol) oder hochmolekularen PEGs (mehr als 2000 g/mol bis 20.000 g/mol) zur Bildung der pharmazeutisch annehmbaren Hülle bevorzugt. In einer besonders bevorzugten Ausführungsform ist die pharmazeutisch annehmbare Hülle bioabbaubar. Eine Vielzahl der obern offenbarten bevorzugten Substanzen und Polymere erfüllen diese Eigenschaft.

Im Stand der Technik ist eine Vielzahl von Verfahren bekannt, die zur Herstellung von umhüllten Eisenkernen geeignet sind. In einigen der Verfahren wird in einem ersten Verfahrensschritt der Eisenkern gebildet und in einem weiteren Schritt die Hülle aufgebracht. Es ist jedoch auch möglich in einer Reaktion bzw. in einer "ein Topf-Reaktion" Eisenkern und Hülle zu bilden. Bekannte Verfahren umfassen ohne Beschränkung das Aerosol/Dampfpyrolyseverfahren, das Gaszersetzungsverfahren, das Sol-Gelverfahren und die Mikroemulsionsverfahren. Besonders bevorzugte Verfahren zur Umhüllung von Eisenoxidkernen sind z.B. in der EP 0 543 020 B1, der EP 0 186 616, der EP 0 525 199 und der EP 0 656 368 beschrieben worden.

Es ist bevorzugt, daß der magnetische Eisenoxidkern Magnetit oder Maghemit oder Gemische davon umfaßt. Es ist möglich, daß dem Eisenoxidkern in bestimmten Ausführungsformen weitere Metalloxide beigemischt sind, die vorzugsweise ausgewählt sind aus Magnesium, Zink und Kobalt. Diese weiteren Metalloxide können in Anteilen von insgesamt bis zu 20% im Eisenoxidkern enthalten sein. In Mengen unter 5%, vorzugsweise unter 1% können des weiteren Mangan, Nickel, Kupfer, Barium, Strontium, Chrom, Lanthan, Gadolinium, Europium, Dysprosium, Holmium, Ytterbium und Samarium enthalten sein.

Die Gesamtdurchmesser der erfindungsgemäßen Partikel hängt vom Durchmesser des Eisenoxidkerns und von der Dicke der diesen umgebenden pharmazeutisch annehmbaren Hülle sowie von weiteren ggf. auf der Hülloberfläche befestigten Molekülen ab. Die obere Grenze des Gesamtdurchmessers wird durch die Maßgabe bestimmt, daß die Partikel nach Applikation in den Körper eines Patienten kapillargängig sein müssen. Die Kapillaren mit dem geringsten Durchmesser finden sich i.d.R. in der Lunge. Diese Kapillaren können von Partikeln mit einem Gesamtdurchmesser von 2 µm noch passiert werden. Erfindungsgemäße Partikel sind vorzugsweise kugelförmig. Allerdings sind längliche, eckige oder im wesentlichen beliebig geformte Partikel auch von der Erfindung umfaßt, solange die Oberfläche des Eisenkerns im wesentlichen durch die pharmazeutisch annehmbare Hülle umhüllt ist. Somit beträgt der Gesamtdurchmesser eines Partikels im Falle eines kugelförmigen Partikels 2r und wird im Falle eines unregelmäßig geformten Partikels durch den Abstand zwischen den zwei auf der Partikeloberfläche liegenden Punkten bestimmt, die am weitesten voneinander entfernt sind, zuzüglich der Dicke der hydratisierten Hülle. Der hier definierte Gesamtdurchmesser ist von dem mittleren Gesamtdurchmesser eines erfindungsgemäßen Partikels zu unterscheiden, der als der durchschnittliche Abstand aller Punkte der Oberfläche des Partikels vom Schwerpunkt des Partikels zuzüglich der Dicke der hydratisierten Hülle definiert ist. Der mittlere Gesamtdurchmesser unterscheidet sich wiederum von dem gemittelten Gesamtdurchmesser der Partikel, der auf eine Gruppe von Partikeln bezug nimmt und sich als Mittelwert aller mittlerer Partikeldurchmesser der in der Gruppe enthaltenden Partikel zuzüglich der hydratisierten Hülle ergibt. Die untere Grenze des Gesamtdurchmessers wird durch die untere Grenze des Durchmessers des Eisenoxidkerns bestimmt, der zu einer verbesserten Bildgebung im MPI führt. In einer bevorzugten Ausführungsform variiert daher der Gesamtpartikeldurchmesser in einem Bereich von ca. 30 nm bis ca. 2 µm. Noch bevorzugter ist jedoch die Verwendung von Partikeln mit einem Gesamtdurchmesser in einem Bereich von ca. 40 bis ca. 500 nm, noch bevorzugter in einem Bereich von ca. 45 nm bis ca. 300 nm, noch bevorzugter in einem Bereich ca. 50 nm bis ca. 200 nm. Der Gesamtdurchmesser der erfindungsgemäßen Partikel läßt sich durch eine Reihe direkter und indirekter im Stand der Technik bekannter Verfahren bestimmen, die bspw. Elektronenmikroskopie und dynamische Lichtstreuung umfassen. Vorzugsweise wird der Gesamtdurchmesser durch dynamische Lichtstreuung bestimmt.

Im verfügbaren Stand der Technik und hierbei vor allem in den bereits erwähnten DE 101 51 778 A1 und DE 102 38 853 A1 werden Partikel beschrieben, die nach Mutmaßung der Anmelder zur Bildgebung beim MPI geeignet sind. Dabei wird lediglich gelehrt, daß Partikel, die einen magnetischen Kern mit einem Durchmesser von 20 bis 800 nm aufweisen, geeignet sind und daß der tatsächlich geeignete Durchmesser des magnetischen Kerns von dem jeweils gewählten magnetischen Material abhängig ist. Eine Lehre zur Verwendung von Partikeln, die Eisenoxidkerne mit einem Durchmesser von 20 nm bis 1 µm bei einem Gesamtpartikeldurchmesser-Kerndurchmesserverhältnis, das kleiner als 6 ist, aufweisen, ist nicht enthalten. Somit sind bevorzugte untere Grenzen des Durchmessers des Eisenoxidkerns 21, 22, 23, 24, 25, 26, 27, 28, 29, 30, 31, 32,33, 34, 35, 36, 37, 38, 39, 40, 41, 42, 43, 44, 45, 46, 47, 48, 49, 50, 5,1, 52, 53, 54, 55, 56, 57, 58, 59, 60, 61, 62, 63, 64, 65, 66, 67, 68, 69, 70, 71, 72, 73, 74, 75, 76, 77, 78, 79, 80, 85, 90, 95, 100 nm. Bevorzugte Obergrenzen des Durchmessers betragen 500, 490, 480, 470, 460, 450, 440, 430, 420, 410, 400, 390, 380, 370, 360, 350, 340, 330, 320, 310, 300, 290, 280, 270, 260, 250, 240, 230, 220, 210, 200, 190, 180, 170, 160, 150, 140, 130, 120, 110, 100, 95, 90, 85 und 80 nm. Alle Kombination der oben genannten Ober- und Untergrenzen sind zur Definition des bevorzugten Bereichs der Partikelgröße möglich, so lange der Wert der Obergrenze größer als der Wert der Untergrenze ist z. B. 40 bis 400 nm, 50 bis 200 nm etc. Bevorzugte erfindungsgemäße Partikel weisen einen Eisenoxidkerndurchmesser in einem Bereich von ca. 25 nm bis ca. 500 nm auf. Noch bevorzugter liegt der Durchmesser des Eisenoxidkerns in einem Bereich von ca. 30 nm bis ca. 200 nm, noch bevorzugter von ca. 35 nm bis ca. 80 nm. Der Durchmesser des Kerns läßt sich wiederum durch im Stand der Technik bekannte Verfahren bestimmen, die ohne Einschränkung auf die genannten Verfahren Röntgenstrukturanalyse und Elektronmikroskopie umfassen. Da die Eisenoxidkerne nicht in allen Fällen eine kugelförmige Gestalt aufweisen, ist der Durchmesser des Eisenoxidkerns der Abstand zwischen den auf der Oberfläche des Eisenoxidkerns gelegenen Punkten, die am weitesten von einander entfernt sind. Dieser Durchmesser ist von dem mittleren Durchmesser eines Eisenoxidkerns zu unterscheiden, der als der durchschnittliche Abstand aller Punkte der Oberfläche des Eisenoxidkerns vom Schwerpunkt des Eisenoxidkerns definiert ist. Der mittlere Durchmesser eines Eisenoxidkems unterscheidet sich wiederum von dem gemittelte Durchmesser des Eisenoxidkerns, der auf eine Gruppe von Partikeln bezug nimmt und sich als Mittelwert aller mittleren Durchmesser der in der Gruppe enthaltenden Eisenoxidkerne ergibt.

Es wurde überraschenderweise beobachtet, daß Partikel, die um den Eisenoxidkern eine dünne Hülle einer pharmazeutisch annehmbaren Substanz aufweisen, für die Bildgebung mit MPI geeigneter sind als Partikel, die bei gleichen Eisenoxidkerndurchmesser eine dickere Hülle aufweisen. In einer bevorzugten Ausführungsform sind die erfindungsgemäßen Partikel daher dadurch gekennzeichnet, daß das Verhältnis des Gesamtpartikeldurchmessers zum Eisenoxidkerndurchmesser kleiner als 6 ist. Noch bevorzugter ist dieses Verhältnis kleiner als 5,5, 5,0, 4,5, 4,0, 3,5, 3,0, 2,9, 2,8, 2,7, 2,6, 2,5, 2,4, 2,3, 2,2, 2,1, 2,0, 1,95, 1,90, 1,85, 1,80, 1,75, 1,60, 1,55 oder kleiner als 1,5. Im Hinblick auf die zuvor genannten Durchmesser der Gesamtpartikel und Eisenoxidkerne weisen besonders bevorzugte Partikel Eisenoxidkerne mit einem Durchmesser im Bereich von ca. 30 bis ca. 200 nm und noch bevorzugter im Bereich von ca. 35 nm bis ca. 80 nm bei einem Verhältnis von Gesamtpartikeldurchmessers zum Eisenoxidkerndurchmesser auf, das kleiner als 3,0, 2,9, 2,8, 2,7, 2,6, 2,5, 2,4, 2,3, 2,2, 2,1, 2,0, 1,95, 1,90, 1,85, 1,80, 1,75, 1,60, 1,55 oder kleiner als 1,5 ist.

Der Begriff "polykristalline magnetische Eisenoxidpartikel" bezeichnet im Zusammenhang mit der vorliegenden Erfindung magnetische Eisenoxidpartikel, die aus mindestens 2 zusammenhängenden Kristallen, vorzugsweise aus 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100 oder mehr Kristallen bestehen. Die maximale Anzahl der Kristalle, die in einem Eisenoxidpartikel der Erfindung enthalten sein kann wird dabei lediglich durch die Größe der Partikel beschränkt. In größeren Partikeln können mehr Kristalle enthalten sein als in kleineren Partikeln. Die Kristalle, die in den polykristallinen magnetischen Eisenoxidpartikeln enthalten sind haben vorzugsweise eine Länge einer Vorzugsrichtung von 1-100 nm, vorzugsweise 3 bis 50 nm. Eine Einheitszelle eines Magnetitkristalls (Fe₃O₄) hat eine Kantenlänge von ca. 1 nm. Dementsprechend weisen die Kristalle, die in den polykristallinen magnetischen Eisenoxidpartikeln enthaltne sind entlang einer Vorzugsrichtung vorzugsweise mindestens 3 Einheitszellen, noch bevorzugter 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60, 65, 70, 75, 80, 85, 90, 95, 100 oder mehr Einheitszellen auf. In den polykristallinen Eisenoxidkernen können an den Grenzflächen zwischen einem oder mehreren Kristallen nicht kristalline Bereich mit ungeordneter amorpher Struktur auftreten, d.h. polykristalline magnetische Eisenoxidkerne bestehen vorzugsweise zu 50%, noch bevorzugter zu 55%, 60%, 65%, 70%, 75%, 80%, 85%, 90%, 95% oder mehr aus polykristallinen Bereichen und der Rest des Eisenoxidpartikels besteht aus ungeordneten amorphen Bereichen. Die einzelnen in dem Eisenoxidkern enthaltenden Kristalle können die gleiche oder verschiedene Längen aufweisen. Der größte Kristallit, d.h. einzelner Kristall, der in den polykristallinen magnetischen Eisenoxidpartikeln enthalten ist, sollte vorzugsweise ein Volumen aufweisen, das kleiner als 70%, noch bevorzugter kleiner als 65%, kleiner als 60%, kleiner als 55%, kleiner als 50%, kleiner als 45% des Gesamtvolumens der erfindungsgemäßen Partikel ist. In einer Ausführungsform der Erfindung besteht keine weitreichweitige Ordnung im polykristallinen Eisenoxidkern, sondern es besteht nur eine kurzreichweitige Ordnung. Dies bedeutet, daß ein im wesentlichen amorpher Partikel mit kristallinen Einschlüssen vorliegt. "Polykristallin" im Sinne der vorliegenden Erfindung umfaßt sowohl Eisenoxidpartikel mit weitreichweitiger als auch kurzreichweitiger Ordnung. Bevorzugt sind Kristalle mit weitreichweitiger Ordnung.

Es sollte darauf hingewiesen werden, daß auch polykristalline Eisenoxidkerne eine Monodomäne ausbilden können und nicht notwendigerweise zur Ausbildung Weiß'scher Bereiche führen. Über die Fähigkeit zur Ausbildung Weiß'scher Bereiche entscheidet in erster Linie die Größe des Eisenoxidkerns. In einer besonders bevorzugten Ausführungsform enthält der Eisenoxidkern mindestens fünf Eisenoxidmonokristalle. Die Anzahl und Größe der in einem Partikel enthaltenden Kristalle kann durch eine Vielzahl verschiedener Verfahren nachgewiesen werden die ohne Beschränkung Transmissionselektronenmikroskopie (TEM), Elektronen-Tomographie und Röntgenbeugung umfassen. Vorzugsweise wird die Größe durch TEM bestimmt.

MPI beruht auf der Detektion der Position magnetischer Partikel. Bei diagnostischen Verfahren, die lediglich auf die Darstellung der inneren Struktur flüssigkeitsdurchströmter Bereiche abzielen, wie bspw. Darstellung des Magen-Darmtrakts oder Darstellung der Koronararterien kann es ausreichen, dem Patienten eine ausreichende Menge der Partikel zuzuführen bspw. durch Injektion einer Partikeldispersion oder durch Schlucken einer geeigneten Partikellösung oder - dispersion. Die Partikel, die im wesentlichen nicht an Strukturen in dem untersuchten Bereich binden, können dann beobachtet werden während sie den untersuchten Bereich durchströmen. Allerdings ist es für viele diagnostische Anwendungen wünschenswert, daß die Partikel eine spezifische Affinität zu Oberflächenstrukturen der untersuchten Bereiche aufweisen. Daher umfaßt das erfindungsgemäße Partikel in einer bevorzugten Ausführungsform auf seiner Oberfläche ein oder mehrere gleiche oder verschiedene Liganden. Die Liganden können kovalent oder nicht kovalent an die Oberfläche gebunden sein. Ein "Ligand" im Sinne der vorliegenden Erfindung ist eine Substanz, die mit einem IC₅₀ von weniger als 10 µM, vorzugsweise von weniger als 1 µM, weniger als 900 nM, weniger als 800 nM, weniger als 700 nM, weniger als 600 nM, weniger als 500 nM, weniger als 400 nM, weniger als 300 nM, weniger als 200 nM, weniger als 100 nM, weniger als 90 nM, weniger als 80 nM, weniger als 70 nM, weniger als 50 nM, weniger als 40 nM , weniger als 30 nM, weniger als 20 nM an eine gegebene Substanz bindet. Im Stand der Technik ist eine Vielzahl von Verfahren bekannt, um die Bindungsaffinität eines Liganden (IC₅₀ oder andere Parameter) an eine gegebene Substanz zu bestimmen. Diese Verfahren umfassen ohne Beschränkung ELISA, Oberflächenplasmonresonanz und Radioliganden-Bindungstestverfahren wie bspw. in Gazal S. et al (2002) J. Med. Chem. 45: 1665-1671 beschrieben.

In einigen Fällen kann es wünschenswert sein zwei oder mehr verschiedene Liganden auf der Oberfläche eines erfindungsgemäßen Partikels zu immobilisieren. Dies bietet sich immer dann an, wenn bspw. auf der Oberfläche an die das Partikel spezifisch binden soll zwei für diese Oberfläche charakteristische Strukturen benachbart vorhanden sind. Dies kann bspw. in bestimmten Tumorzellen der Fall sein bei denen auf Grund einer Mutation zwei Komponenten eines Rezeptors dauerhaft miteinander verbunden sind. Die Verwendung von zwei Liganden, die jeweils gegen eine der beiden Strukturen gerichtet sind, führt dann zu einer deutlichen Affinitäts- bzw. Spezifitätserhöhung. In dieser bevorzugten Ausgestaltung der erfindungsgemäßen Partikel wird (werden) auf der Oberfläche der Hülle, die aus einer pharmazeutisch annehmbaren Substanz besteht, zusätzlich eine oder mehrere Ligandensorten immobilisiert. Abhängig von der jeweiligen Substanz bzw. Substanzmischung aus der die pharmazeutisch annehmbare Hülle besteht kann diese selbst eine Affinität für eine gegebene Substanz aufweisen. Bspw. weisen eine Reihe von Polysacchariden eine gewisse Zellspezifität auf. Insofern erfordert die Herstellung von z.B. zellspezifischen Partikeln nicht in jedem Fall die Immobilisierung von Liganden, da sich eine solche Affinität auch aus der pharmazeutisch annehmbaren Hülle ergeben kann.

Die Anzahl der maximal auf der Oberfläche immobilisierbaren Liganden wird durch die Größe der Oberfläche und den Platzbedarf des jeweiligen Liganden bestimmt. In der Regel wird der Ligand in einer monomolekularen Schicht an der Oberfläche des Partikels gebunden, was je nach Größe des Liganden zu einer signifikanten Vergrößerung des Durchmessers führen kann. Bei der Auswahl geeigneter Liganden und insbesondere bei der Verwendung großer Antikörperliganden muß daher darauf geachtet werden, daß der Gesamtdurchmesser des sich ergebenden Ligandenbeschichteten Partikels nicht dazu führt, daß das Partikel nicht mehr dazu in der Lage ist Kapillaren zu passieren. Es ist daher bevorzugt, daß der Durchmesser des Ligandenbeschichteten Partikels unter 2 µm liegt und noch bevorzugter einen der oben im Hinblick auf den Gesamtdurchmesser des unbeschichteten Partikels angegebenen bevorzugten Durchmesser aufweist. In diesem Fall muß die Dicke der pharmazeutisch annehmbaren Hülle und/oder der Durchmesser des Eisenoxidkerns entsprechend verringert werden. Im Stand der Technik ist eine Vielzahl von Verfahren zur Befestigung von Liganden offenbart. Besonders bevorzugte Verfahren sind bspw. in der US 6,048,515 offenbart. Abhängig von der jeweiligen pharmazeutisch annehmbaren Hülle kann es erforderlich sein, die Hülle vor der Befestigung des Liganden querzuvernetzen. Solche Verfahren sind z.B. durch Weißleder et al. beschrieben worden.

Die Auswahl des Liganden wird von der Krankheit bzw. dem Zustand abhängen, der durch MPI der Diagnose zugänglich gemacht werden soll. Vorzugsweise liegen die Strukturen an die die auf den Partikeln befindlichen Liganden binden, in den von Körperflüssigkeiten, z.B. Blut oder Lymphe umspülten Bereichen bzw. in den Körperflüssigkeiten enthalten sind. Es ist daher bevorzugt, daß der Ligand dazu in der Lage ist, spezifisch an zelluläre (eukaryotische oder prokaryotische), extrazelluläre oder virale Oberflächenstrukturen zu binden. Im Stand der Technik ist inzwischen eine Vielzahl von Strukturen bekannt, die präferentiell in erkrankten Geweben oder Zellen oder in der Nähe solcher Gewebe oder Zellen exprimiert werden und die daher als Hinweis auf die jeweilige Krankheit dienen können. Beispielsweise ist die Neubildung von Blutgefäßen (Neoangiogenese) im adulten Körper auf die Gebärmutterschleimhaut im Zusammenhang mit Menstruation oder Schwangerschaft und auf Heilungsprozesse im Anschluß an gefäßverletzende Traumata beschränkt. Inzwischen ist allerdings bekannt, daß neue Blutgefäße auch bei einer Vielzahl proliferativer Erkrankungen gebildet werden, die sonst an keiner anderen Stelle des Körpers anzutreffen sind, die nicht von der proliferativen Erkrankung betroffen ist. Daher sind zelluläre Strukturen, die im Zusammenhang mit der Neoangiogenese stehen, und insbesondere Strukturen, die nur auf Tumorendothel gefunden werden, wie bspw. die ED-B-Domäne des Fibronectins (ED-BF), hervorragende Ziele für die Liganden, die auf der Oberfläche der erfindungsgemäßen Partikel immobilisiert werden können. Alle im Stand der Technik bekannten Liganden gegen solche krankheitsassoziierten Strukturen können im Zusammenhang mit den Partikeln der vorliegenden Erfindung verwendet werden. Besonders bevorzugte Liganden sind jedoch die Liganden, die dazu in der Lage sind spezifisch an eine der folgenden Stukturen zu binden: an die ED-B-Domäne des Fibronectins (ED-BF), an Endoglin, an den vaskulären entothelialen Wachstumsfaktorrezeptor (VEGFR), an VEGF Familienmitglieder, an NRP-1, an Ang1, an Thie2, an PDGF-BB und Rezeptoren, an TGF-β1, an TGF-β Rezeptoren, an FGF, an HGF, an MCP-1, an Integrine (αᵥβ₃, αᵥβ₅, α₅β₁), an VE-Cadherine, an PECAM (CD31), an Ephrine, an Plasminogenaktivatoren, an MMPs, an PAI-1, an NOS, an COX-2, an AC133, an Chemokine, an Id1/Id3, an VEGFR-1, an Ang2, an TSP-1,-2, an Angiostatine und verwandte Plasminogen-Kringel, an Endostatine (Collagen-XVII-Fragment), an Vasostatin, an Bluttplättchenfaktor 4 (PF4), an TIMPs, an MMP-Inhibitoren, an PEX, an Meth-1, an Meth-2, an IFN-α, -β, -y, an IL-10, an IL-4, an IL-12, an IL-18, an Prolactin (M, 16K), an VEGI, an Fragmente von SPARC, an Osteopontin Fragmente oder Maspin, an CollXVIII, an CM201, an Statine, insbesondere L Statin, an CD105, an ICAM1, an Somatostatin (Subtyp 1, 2, 3, 4, oder 5) oder Somatostatinrezeptoren (Subtyp 1, 2, 3, 4, 5, oder 6).

Es ist im Stand der Technik bekannt, daß eine Vielzahl von Substanzen eine Affinität vorzugsweise gegenüber zellulären (eukaryotischen oder prokaryotischen), extrazellulären oder viralen Oberflächenstrukturen aufweisen. Vorzugsweise sind die Liganden, die auf den erfindungsgemäßen Partikeln immobilisiert werden, ausgewählt aus einem Polypeptid, einem Oligonukleotid, einem Polysaccharid und einem Lipid.

Polypeptide, die eine spezifische Affinität aufweisen, sind bekannt und lassen sich durch eine Reihe von Verfahren identifizieren die "Phage-Display" und Immunisierung umfassen. Es ist in diesem Zusammenhang bevorzugt, daß die erfindungsgemäß als Liganden verwendbaren Proteine ausgewählt sind aus der Gruppe bestehend aus einem Antikörper, umfassend humane, humanisierte, und chimäre Antikörper und Antikörperfragemente, Antikörperbindungsdomänenumfassende Fragmente, z.B. Fv, Fab, Fab', F(ab')₂, Fabc, Facb, einzelkettige Antikörper, z.B. single chain Fvs (scFvs) und Diabodies, und einem Liganden eines zellulären, extrazellulären oder viralen Rezeptors oder eines Fragments davon. Geeignete Liganden sind beispielsweise der Vascular Endothelial Growth Factor (VEGF), Epidermal Growth Factor (EGF), Chemokine oder Cytokine.

Die Fähigkeit von Nukleinsäuren spezifische Bindungen bspw. mit Transkriptionsfaktoren oder Histonen einzugehen ist gut bekannt. Bevorzugte Oligonukleotide, die als Liganden auf den erfindungsgemäßen Partikeln immobilisiert werden können, umfassen DNA, RNA, PNA und Aptamere. PNAs sind dabei besonders bevorzugt, da sie eine höhere Beständigkeit gegenüber den üblicherweise im Patienten anzutreffenden Nukleasen und damit ein längere biologische Halbwertzeit aufweisen. Verfahren zur Identifikation spezifisch bindender Nukleinsäuren sind im Stand der Technik bekannt und sind bspw. in WO 93/24508 A1, WO 94/08050 A1, WO 95/07364 A1, WO 96/27605 A1 und WO 96/34875 A1 beschrieben.

In einigen Fällen wird es bspw. aus sterischen oder aus Gründen der chemischen Inkompatibilität nicht möglich sein, den Liganden direkt an die Oberfläche des Partikels zu binden. In diesen Fällen kann der Ligand mit der Oberfläche des Partikels über einen Linker verbunden sein. Der Begriff "Linker" bezeichnet in diesem Zusammenhang ein Molekül, das vorzugsweise eine oder zwei chemisch reaktive Gruppen aufweist, die jeweils die kovalente oder nich-kovalente Kopplung an die Partikeloberfläche einerseits und an den Liganden andererseits erlauben. Zwischen diesen Kopplungsgruppen ist in der Regel ein linearer, cyclischer oder verzweigter Bereich angeordnet, der bspw. eine größere räumliche Trennung zwischen Ligand und Partikel bzw. eine höhere Beweglichkeit des Liganden ermöglicht. Dieser lineare Bereich kann bspw. eine substituierte oder unsubstituierte, verzweigte oder unverzweigte, gesättigte oder ungesättigte Alkylkette (C₂ bis C₅₀) sein, die durch eine oder mehrere O-, N- und/oder S-Atome unterbrochen sein kann, ein Polpeptid oder ein Polynukleotide sein. Beispiele chemisch reaktiver Gruppen, die in diesen Linkern eingesetzt werden können, umfassen bspw. Amino-, Hydroxyl-, Thiol-, oder Thiol-reaktive-, Sulfhyddryl-, Carboxyl-, und Epoxidgruppen. Thiol-reaktive Gruppen umfassen z.B. Maleinimid- (Maleimid), Chloracetyl-, Bromacetyl-, Iodacetyl-, Chloracetamido-, Bromacetamido-, Iodacetamido-, Chloralkyl-, Bromalkyl-, Iodalkyl-, Pyridyldisulfid- and Vinylsulfonamidgruppen. Eine Vielzahl von Kopplungsreagenzien, Kopplungsgruppen sowie Linker sind in der WO 98/47541 offenbart, auf die hier spezifisch im Hinblick auf diese Offenbarung verwiesen wird.

Es ist beobachtet worden, daß Polyethylenglycolreste (PEG) und/oder Polypropylenglycolreste (PPG), die auf der Oberfläche pharmazeutischen Wirkstoffe immobilisiert sind, zu einer deutlichen Verlängerung der biologischen Halbwertzeit führen. Beispiele dafür sind PEGylierte Liposomen oder PEGylierte Proteine, wie bspw. PEGyliertes EPO. Verfahren zur PEGylierung von Oberflächen sind im Stand der Technik wohl bekannt. Abhängig von dem jeweiliges verwendeten, pharmazeutisch annehmbaren Hüllstoff können die PEG-Reste oder die PPG-Reste direkt auf der Oberfläche oder über einen Linker kovalent oder nicht-kovalent immobilisiert werden. Bevorzugt sind die Polyethylenglycol- und/oder Polypropylenglycolreste nicht-kovalent mit der Oberfläche des Partikels verbunden.

Ein weiterer Gegenstand der vorliegenden Erfindung ist ein Verfahren zur Herstellung von Partikeln, die in einer pharmazeutisch annehmbaren Hülle einen magnetischen Eisenoxidkern mit einem Durchmesser von 20 nm bis 1 µm umfassen, deren Gesamtpartikeldurchmesser-Kerndurchmesserverhältnis kleiner als 6 ist, umfassend die folgenden Schritte:
(i) Mischen einer wäßrigen Mischeisensalzlösung, die ein Eisen-(II)-Salz und eine Eisen-(III)-Salz enthält, mit einer Base in Gegenwart eines synthetischen Polymers oder Copolymers, einer Stärke oder eines Derivats davon, eines Dextrans oder ein Derivats davon, eines Cyclodextrans oder Derivats davon, einer Fettsäure, eines Polysaccharid, eines Lecithins oder eines Mono-, Di- oder Triglycerids oder eines Derivats davon oder Gemischen davon, bis zur Bildung einer kolloidalen Lösung der Partikel,
(ii) Durchleiten der Partikel-haltigen Lösung durch ein magnetisches Gradientenfeld,
(iii) Entfernung des magnetischen Gradientenfelds
(iv) und Gewinnung der im Gradientenfeld zurückgehaltenen Partikel.

Schritt (i) des erfindungsgemäßen Verfahren führt in der Regel nicht zu einer homogenen Gruppe von Partikeln sondern zu einer Gruppe von Partikeln, die sowohl hinsichtlich des Durchmessers des Eisenoxidkerns als auch hinsichtlich des Durchmessers des Gesamtpartikels innerhalb bestimmter Bandbreiten variieren. Der gemittelte Eisenoxidkerndurchmesser einer solchen Gruppe ergibt sich als Mittelwert aller in der Gruppe enthaltender mittlerer Eisenoxidkerndurchmesser. Die Anlegung des magnetischen Gradientenfelds des Schritts (ii) führt zur Selektion von Partikeln mit einem im Vergleich zum gemittelten Eisenoxidkerndurchmesser größeren Eisenoxidkern. So lassen sich durch die Auswahl der Stärke des magnetischen Gradientenfelds und ggf. durch ein, zwei oder mehrmalige Wiederholung der Schritte (ii) bis (iv) des erfindungsgemäßen Verfahrens ggf. unter Veränderung der Stärke des Gradientenfelds aus einer heterogenen Gruppe von Partikeln eine Partikelgruppe auswählen, die einen im Vergleich zum gemittelten Eisenoxidkerndurchmesser größeren gemittelten Eisenoxidkerndurchmesser aufweisen und eine dünnere Hülle aus pharmazeutisch annehmbaren Materialien. I.d.R. wird am Ende der Schritte (ii) bis (iv) auch eine Partikelgruppe gewonnen, die eine geringere Streuung der Kerndurchmesser aufweist.

Um aus dieser Untergruppe von Partikeln die Partikel auszuwählen, die mindestens einen vorbestimmten Gesamtpartikeldurchmessers aufweisen, können diese durch im Stand der Technik bekannte Verfahren wie Filtration, Sedimentation, Gegenstromzentrifugation (Elutriation) etc. ausgewählt werden.

Die in dem erfindungsgemäßen Verfahren verwendeten Eisen-(II)- und Eisen-(III)-Salze liegen vorzugsweise in wäßriger Lösung in Konzentration von 0,1 bis 2 M vor. Die divalenten und trivalente Eisenionen liegen vorzugsweise in einem Mischungsverhältnis von 1:3 bis 2:1 vor. Geeignete Anionen der Eisensalze sind aus organischen Säuren abgeleitet wie bspw. aus Zitronensäure, Milchsäure, Essigsäure, Maleinsäure etc. oder aus anorganischen Säuren wie bspw. aus HCl, H₂SO₄, H₂SO₃, HBr, HI, HNO₃ oder HNO₂.

Die Base ist vorzugsweise ausgewählt aus anorganischen Basen, wie z.B. NaOH, KOH, LiOH oder Al(OH)₃ und aus organischen Basen. Die Base kann der wäßrigen Reaktionslösung als Feststoff oder als Lösung, vorzugsweise als wäßrige Lösung zugesetzt werden. Dieser Zusatz erfolgt vorzugsweise bis die Lösung einen pH-Wert von 10 vorzugsweise 11 oder höher erreicht. An diesen Schritt der Basifizierung schließt sich vorzugsweise eine Neutralisierung durch Säure, vorzugsweise HCl auf einen pH von ungefähr 7 ± 0,5 an.

Als weiteren Schritt kann sich an den Schritt (i) unmittelbar oder nach erfolgter Neutralisierung eine Wärmebehandlung anschließen. Hierbei wird die Lösung auf mindestens 50°C erwärmt, vorzugsweise auf 60°C, 65°C, 70°C, 75°C, 80°C, 90°C oder 95°C. Falls eine Neutralisierung noch nicht stattgefunden hat, kann die Lösung nach der Erhitzung durch Zugabe von Säure neutralisiert werden und dann entweder abgekühlt oder weiter erhitzt werden. Wenn die Lösung weiter erhitzt wird, wird sie vorzugsweise auf 50°C, vorzugsweise auf 60°C, 65°C, 70°C, 75°C, 80°C, 85°C 90°C, 95°C, 100°C oder bis zum Rückfluß erhitzt. Eine solche Erhitzung findet vorzugsweise für 10 min bis 10 h statt.

Unmittelbar im Anschluß an Schritt (i) oder auch im Anschluß an jeden der vorangehend beschriebenen weiteren Schritte, d.h. den Neutralisierungs- und/oder Erwärmungsschritt können sich ein oder mehrere Wasch- und/oder Dialyseschritte anschließen. Des weiteren ist es bevorzugt, daß die erhaltenen Partikel im Anschluß an Schritt (iv) einem oder mehreren Wasch-und Dialyseschritten unterzogen wird. Das Ziel dieses(r) Schritts(e) liegt vorzugsweise darin potentiell schädliche Verunreinigung, die aus dem Herstellungsverfahren zurückgeblieben sind, von den Partikeln abzutrennen sowie den pH und oder den Salzgehalt einer die Partikel enthaltenden Lösung anzupassen.

Das magnetische Gradientenfeld dient der Auswahl von Teilchen, die besonders für die MPI-Verfahren geeignet sind. Das magnetische Gradientenfeld, das für das erfindungsgemäße Verfahren eingesetzt werden kann, kann stark variieren und kann vom Fachmann unter Berücksichtigung verschiedener Parameter der Versuchsanordnung eingestellt werden. Das zur Separation verwendete Gradientenfeld muß deutlich größer als der Gradient des Erdfeldes sein. Das magnetische Gradientenfeld im Separationsraum kann durch ein permanent-magnetisches Material oder durch einen Strom-durchflossenen Leiter erzeugt werden. Im Zusammenhang mit der vorliegenden Erfindung ist die letztere Ausgestaltung bevorzugt, da sie eine leichte Entfernung des Gradientenfelds durch Abschalten des Stroms erlaubt. Im ersteren Fall erfolgt die Entfernung des magnetischen Gradientenfelds durch Entfernung des permanent-magnetischen Materials. Das Gradientenfeld liegt typischerweise im Bereich von 1 mT/m bis 5000 T/m. Sollen beispielsweise Partikel mit >100 nm Kerndurchmesser zurückgehalten werden und wird Schritt (ii) bei einer Anordnung mit geringer Durchflußgeschwindigkeit durchgeführt, so ist es bevorzugt Gradientenstärken von 1-10 mT/m anzuwenden. Sollen jedoch Partikel mit kleinerem bspw. ca. 20 nm Kerndurchmesser zurückgehalten werden und wird das Verfahren mit hoher Durchflußgeschwindigkeit durchgeführt, so ist es bevorzugt Gradientenstärken > 1000 T/m zu verwenden. Das Ziel des Anlegen eines Gradientenfelds besteht in der Anreicherung von solchen Partikeln, die einen größeren Eisenoxidkerndurchmesser aufweisen und/oder die ein bevorzugtes, d.h. niedriges Gesamtdurchmesser/Kernverhältnis aufweisen. Der Fachmann kann unter Berücksichtigung der hier zur Verfügung gestellten Lehre und abhängig von dem gemittelten Durchmesser, der mit dem erfindungsgemäßen Verfahren hergestellten Partikel und der für das Durchleiten gewählten Strömungsgeschwindigkeit, eine geeignete Gradientenfeldstärke bestimmen, die die Auswahl solcher Partikel ermöglicht. Geeignete, mit einem magnetischen Gradientenfeld versehene Separationsvorrichtungen sind bspw. unten in den Beispielen 1b) und 1c) und in der EP 0 915 738 B1 beschrieben. Die Vorrichtung können in dem Bereich des Gradientenfelds weitere paramagnetische Materialien enthalten, um ggf. das Gradientenfeld zu verstärken.

Zur Gewinnung der im Gradientenfeld zurückgehaltenen Partikel wird die Separationsvorrichtung vorzugsweise nach Entfernung des Gradientenfelds mit einer geeigneten Lösung, vorzugsweise einer wäßrigen pharmazeutisch annehmbaren Lösung durchspült, wobei die Partikel vorzugsweise mechanisch aus der Separationsvorrichtung gelöst werden.

Die erfindungsgemäß hergestellten Partikel weisen vorzugsweise eine T₂-Relaxivität von mindestens 150 (mM • s)⁻¹, noch bevorzugter, von mindestens ca. , 160,170,180,190, 200, 210, 220, 230, 240, 250, 260, 270, 280, 290, 300, 310, 320, 330, 340, 350, 400, 450, 500, 550, 600, 650, 700, 750, 800, 850, 900, 950 oder 1000 (mM · s)⁻¹ auf. Die T₂-Relaxivität der Partikel wird vorzugsweise bei einer Magnetfeldstärke von 1 Tesla in einer wäßrigen kolloidalen Lösung der Partikel bestimmt. Geeignete Meßverfahren sind dem Fachmann bekannt und sind z.B. auch in den angefügten Beispielen offenbart. In einer bevorzugten Ausführungsform des erfindungsgemäßen Verfahrens variiert der Gesamtpartikeldurchmesser in einem Bereich von ca. 30 nm bis ca. 2 µm. Noch bevorzugter ist jedoch die Verwendung von Partikeln mit einem Gesamtdurchmesser in einem Bereich von ca. 40 bis ca. 500 nm, noch bevorzugter in einem Bereich von ca. 45 nm bis ca. 300 nm, noch bevorzugter in einem Bereich ca. 50 nm bis ca. 200 nm. Der Gesamtdurchmesser der erfindungsgemäßen Partikel läßt sich wie bereits erwähnt durch eine Reihe direkter und indirekter im Stand der Technik bekannter Verfahren bestimmen, die bspw. Elektronenmikroskopie und dynamische Lichtstreuung umfassen. Bevorzugte untere Grenzen des Durchmessers des im Partikel enthaltenden Eisenoxidkerns betragen 21, 22, 23, 24, 25, 26, 27, 28, 29, 30, 31, 32,33, 34, 35, 36, 37, 38, 39, 40, 41, 42, 43, 44, 45, 46, 47, 48, 49, 50, 5,1, 52, 53, 54, 55, 56, 57, 58, 59, 60, 61, 62, 63, 64, 65, 66, 67, 68, 69, 70, 71, 72, 73, 74, 75, 76, 77, 78, 79, 80, 85, 90, 95, 100 nm. Bevorzugte Obergrenzen des Durchmessers betragen 500, 490, 480, 470, 460, 450, 440, 430, 420, 410, 400, 390, 380, 370, 360, 350, 340, 330, 320, 310, 300, 290, 280, 270, 260, 250, 240, 230, 220, 210, 200, 190, 180, 170, 160, 150, 140, 130, 120, 110, 100, 95, 90, 85 und 80 nm. Alle Kombination der oben genannten Ober- und Untergrenzen sind zur Definition des bevorzugten Bereichs der mit dem erfindungsgemäßen Verfahren herstellbaren Partikelgrößen möglich, so lange der Wert der Obergrenze größer als der Wert der Untergrenze ist z. B. 40 bis 400 nm, 50 bis 200 nm etc. Bevorzugte erfindungsgemäß hergestellte Partikel weisen einen Eisenoxidkerndurchmesser in einem Bereich von ca. 25 nm bis ca. 500 nm auf. Noch bevorzugter liegt der Durchmesser des Eisenoxidkerns in einem Bereich von ca. 30 nm bis ca. 200 nm, noch bevorzugter von ca. 35 nm bis ca. 80 nm.

Ein weiterer Gegenstand der vorliegenden Erfindung sind Partikel, die mit einem erfindungsgemäßen Verfahren herstellbar sind.

Ein weiterer Gegenstand der vorliegenden Erfindung ist eine Zusammensetzung, wobei mindestens 2% der in der Zusammensetzung enthaltenden Partikel, die in einer pharmazeutisch annehmbaren Hülle einen magnetischen Eisenoxidkern enthalten, erfindungsgemäße Partikel oder Partikel sind, die nach dem erfindungsgemäßen Verfahren hergestellt werden. In bevorzugten Ausgestaltungen ist der Anteil der erfindungsgemäßen bzw. erfindungsgemäß hergestellten Partikel höher und beträgt mindestens 5%, 10%, 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% oder 100%. Die Eignung der erfindungsgemäßen Zusammensetzungen für MPI-Verfahren steigt mit dem Anteil der erfindungsgemäßen Partikel bzw. der erfindungsgemäßen hergestellten Partikel.

Ein weiterer Gegenstand der vorliegenden Erfindung ist eine Zusammensetzung, wobei der Partikeldurchmesser der erfindungsgemäßen Partikel oder der nach dem erfindungsgemäßen Verfahren hergestellten Partikel für mindestens 50% der Partikel in einem Bereich von 10% um den gemittelten Partikelgrößendurchmesser liegt. Eine Erhöhung der Homogenität der Größenverteilung und damit des von dem jeweiligen Partikel erzeugten Signals ist bevorzugt. In diesem Zusammenhang ist es besonders bevorzugt, wenn mindestens 55% mindestens 60%, 65%, 70%, 75%, 80%, 90%, 95% der erfindungsgemäßen Partikel bzw. nach dem erfindungsgemäßen Verfahren hergestellte Partikel einen Partikeldurchmesser aufweisen, der in einem Bereich von 10% um den gemittelten Partikeldurchmesser liegt. Der gemittelte Partikeldurchmesser hat in diesem Zusammenhang die bereits erwähnte Bedeutung, d.h. der Partikeldurchmesser ist der gemittelte Durchmesser aller in der Zusammensetzung befindlichen erfindungsgemäßen bzw. erfindungsgemäß hergestellten Partikel.

Ein weiterer Gegenstand der vorliegenden Erfindung ist eine Flüssigkeit, die eine erfindungsgemäße Zusammensetzung enthält. Geeignete Flüssigkeiten sind wäßrige vorzugsweise auf einen physiologischen pH gepufferte Lösungen, die ggf. Salze, Zucker etc. enthalten, insbesondere wenn sie zur parenteralen Applikation vorgesehen sind. Die Flüssigkeiten enthalten ggf. Zusatzstoffe wie Konservierungsmittel, Stabilisatoren, Detergenzien, Aromastoffe, Träger etc. Dem Fachmann ist eine Vielzahl von Stoffen bekannt, die je nach Applikationsweg diagnostischen Lösungen beigefügt werden können. Diese Zusatzstoffe können den erfindungsgemäßen Flüssigkeiten ohne Ausnahme zugefügt werden, es sei denn, es ergeben sich Inkompatibilitäten mit den erfindungsgemäßen Partikeln. Dabei ist bevorzugt, daß die Flüssigkeit in Form einer stabilisierten kolloidalen Lösung vorliegt.

Ein weiterer Gegenstand der vorliegenden Erfindung ist die Verwendung einer erfindungsgemäßen Zusammensetzung oder einer erfindungsgemäßen Flüssigkeit zur Herstellung eines Diagnostikums für die Verwendung bei Bildgebung durch Detektion magnetischer Partikel (MPI) zur Diagnose von Erkrankungen. Es ist dabei bevorzugt, daß die Erkrankungen ausgewählt sind aus der Gruppe bestehend aus proliferativen Erkrankungen, insbesondere Tumore und Metastasen, inflammatorischen Erkrankungen, Autoimmunerkrankungen, Erkrankungen des Verdauungstrakts, Arteriosklerose, Schlaganfall, Infarkt, krankhafte Veränderungen des Blutgefäßsystems, des Lymphsystems, der Bauchspeicheldrüse, der Leber, der Niere, des Gehirns und der Blase, sowie Erkrankungen der elektrischen Reizweiterleitung und neurodegenerative Erkrankungen. Die in dieser Verwendung eingesetzten Partikel sind vorzugsweise die vorangehend offenbarten bevorzugten oder besonders bevorzugten Partikel.

Ein weiterer Gegenstand der vorliegenden Erfindung ist die Verwendung einer Zusammensetzung, wobei mindestens 2% der in der Zusammensetzung enthaltenden Partikel in einer pharmazeutisch annehmbaren Hülle einen magnetischen Eisenoxidkern mit einem Durchmesser von 20 nm bis 1 µm umfassen, deren Gesamtpartikeldurchmesser-Kerndurchmesserverhältnis kleiner als 6 ist, zur Herstellung eines Diagnostikums für die Verwendung bei der Bildgebung durch Detektion magnetischer Partikel (MPI) zur Diagnose von Erkrankungen. Es ist dabei bevorzugt, daß die Erkrankungen ausgewählt sind aus der Gruppe bestehend aus proliferativen Erkrankungen, insbesondere Tumore und Metastasen, inflammatorischen Erkrankungen, Autoimmunerkrankungen, Erkrankungen des Verdauungstrakts, Arteriosklerose, Schlaganfall, Infarkt, krankhafte Veränderungen des Blutgefäßsystems, des Lymphsystems, der Bauchspeicheldrüse, der Leber, der Niere, des Gehirns und der Blase, sowie Erkrankungen der elektrischen Reizweiterleitung und neurodegenerative Erkrankungen. Bevorzugte Eisenoxidkerndurchmesser dieser Partikel sind die vorangehend genannten Durchmesser umfassen aber auch Eisenoxidkerne mit einem Durchmesser von mindestens 5 nm, mindestens 6, 7, 8, 9, 10, 11, 12, 13, 14, 15, 16, 17, 18, 19, 20, 21, 22, 23, 24, 25, 26, 27, 28, 29 und 30 nm. Die bei dieser erfindungsgemäßen Verwendung verwendbaren Partikel können mit den vorangehend dargestellten Verfahren erzeugt werden.

Es ist dabei besonders bevorzugt, daß die Detektion der Partikel durch ein Verfahren umfassend die folgenden Schritte erfolgt:
(i) Erzeugung eines Magnetfeldes mit einem solchen räumlichen Verlauf der magnetischen Feldstärke, daß sich in dem Untersuchungsbereich ein erster Teilbereich mit niedriger magnetischer Feldstärke und ein zweiter Teilbereich mit höherer magnetischer Feldstärke ergibt,
(ii) Veränderung der räumlichen Lage der beiden Teilbereiche in dem Untersuchungsbereich, so daß sich die Magnetisierung der Partikel örtlich ändert,
(iii) Erfassung von Signalen, die von der durch diese Veränderung beeinflußten Magnetisierung im Untersuchungsbereich abhängen,
(iv) Auswertung der Signale zur Gewinnung von Information über die räumliche Verteilung der magnetischen Partikel im Untersuchungsbereich.

Bei diesem Verfahren und bei dieser Anordnung wird im Untersuchungsbereich ein räumlich inhomogenes Magnetfeld erzeugt. In dem ersten Teilbereich ist das Magnetfeld so schwach, daß die Magnetisierung der Partikel mehr oder weniger stark vom äußeren Magnetfeld abweicht, also nicht gesättigt ist. In dem zweiten Teilbereich (d.h. in dem außerhalb des ersten Teils verbleibenden Rest des Untersuchungsbereichs) ist das Magnetfeld genügend stark, um die Partikel in einem Zustand der Sättigung zu halten. Die Magnetisierung ist gesättigt, wenn die Magnetisierung nahezu aller Partikel in ungefähr der Richtung des äußeren Magnetfeldes ausgerichtet ist, so daß mit einer weiteren Erhöhung des Magnetfeldes die Magnetisierung dort wesentlich weniger zunimmt als im ersten Teilbereich bei einer entsprechenden Erhöhung des Magnetfeldes.

Der erste Teilbereich ist vorzugsweise ein räumlich zusammenhängender Bereich; er kann ein punktförmiger Bereich sein, aber auch eine Linie oder eine Fläche. Entsprechend einer Ausgestaltung wird der erste Teilbereich von dem zweiten Teilbereich räumlich umschlossen.

Durch Veränderung der Lage der beiden Teilbereiche innerhalb des Untersuchungsbereichs ändert sich die (Gesamt-)Magnetisierung im Untersuchungsbereich. Die Veränderung der räumlichen Lage der Teilbereiche kann beispielsweise mittels eines zeitlich veränderlichen Magnetfeldes vonstatten gehen. Mißt man die Magnetisierung im Untersuchungsbereich oder davon beeinflußte physikalische Parameter, dann kann man daraus Informationen über die räumliche Verteilung der magnetischen Partikel im Untersuchungsbereich ableiten.

Dazu werden beispielsweise die durch die zeitliche Änderung der Magnetisierung im Untersuchungsbereich in wenigstens einer Spule induzierten Signale empfangen und ausgewertet. Wirkt ein zeitlich veränderliches Magnetfeld in einem ersten Frequenzband auf den Untersuchungsbereich bzw. auf die Partikel ein, so werden aus den von der Spule empfangenen Signalen nur jene Signale ausgewertet, die eine oder mehrere höhere Frequenzkomponenten als die des ersten Frequenzbandes enthalten. Diese gemessenen Signale entstehen dadurch, daß die Magnetisierungskennlinie der Partikel in der Regel nicht linear verläuft.

Für weitere Erläuterungen zu dem Verfahren und der Anordnung wird auf die DE 101 51 778 verwiesen. Da hier das Verfahren und die Anordnung in aller Ausführlichkeit beschrieben sind, wird an dieser Stelle ausdrücklich der Inhalt der DE 101 51 778 in vollem Umfang einbezogen (incorporate by reference).

In einer bevorzugten Ausführungsform betrifft die Erfindung die Verwendung einer erfindungsgemäßen Zusammensetzung oder Flüssigkeit, wobei die Anordnung zur Detektion die folgenden Mittel umfaßt:
a) Mittel zum Erzeugen eines Magnetfeldes mit einem solchen räumlichen Verlauf der magnetischen Feldstärke, daß sich in dem Untersuchungsbereich ein erster Teilbereich mit niedriger magnetischer Feldstärke und ein zweiter Teilbereich mit höherer magnetischer Feldstärke ergibt,
b) Mittel zum Verändern der räumlichen Lage der beiden Teilbereiche in dem Untersuchungsbereich, so daß die Magnetisierung der Partikel sich örtlich ändert
c) Mittel zur Erfassung von Signalen, die von der durch die Veränderung der räumlichen Lage beeinflußten Magnetisierung im Untersuchungsbereich abhängen,
d) Mittel zur Auswertung der Signale zur Gewinnung von Information über die räumliche Verteilung der magnetischen Partikel im Untersuchungsbereich.

Da die erfindungsgemäß verwendbaren Teilchen eine besonders hohe räumliche Auflösung ermöglichen, ist die Verwendung zur Diagnose proliferativer Erkrankungen und dabei insbesondere früher Phasen solcher Erkrankungen geeignet. Die proliferative Erkrankung ist vorzugsweise ausgewählt aus der Gruppe bestehend aus einem Tumor, einer Präcancerose, eine Dysplasie, einer Endometriose und einer Metaplasie.

Weitere bevorzugte Erkrankung für deren Diagnose die erfindungsgemäß verwendbaren Partikel eingesetzt werden können, umfassen die Autoimmunerkrankungen, die ausgewählt sind aus der Gruppe bestehend aus rheumatoider Arthritis, inflammatorischer Darmerkrankung, Osteoarthritis, neuropathischen Schmerzen Alopecia areta, Psoriasis, psoriatische Arthritis, akute Pancreatitis, Allograftabstoßung, Allergien, allergische Entzündungen der Lunge, Multiple Sclerose, Alzheimer, Morbus Crohn, und systemischer Lupus erythematosus.

Die erfindungsgemäßen und erfindungsgemäß hergestellten Partikel können in einer anderen Ausführungsform der vorliegenden Erfindung in einem Verfahren zur lokalen Erwärmung mit magnetischen Partikeln eingesetzt werden, besonders bevorzugt zur Erzeugung von Hyperthermie eingesetzt werden. Für weitere Erläuterungen zu dem Verfahren und der Anordnung wird auf die WO2004/018039 verwiesen. Da hier das Verfahren und die Anordnung in aller Ausführlichkeit beschrieben sind, wird an dieser Stelle ausdrücklich der Inhalt der WO2004/018039 in vollem Umfang einbezogen (incorporate by reference).

In einer weiteren Ausführungsform können die neuen erfindungsgemäßen bzw. erfindungsgemäß hergestellten Partikel als T₂ verstärkende Kontrastmittel zum Magnetic Resonance Imaging (MRI) eingesetzt werden. Die Partikel als MRI Kontrastmittel zur Diagnose von Erkrankungen und krankhaften Veränderungen ausgewählt aus der Gruppe bestehend aus proliferativen Erkrankungen, inflammatorischen Erkrankungen, Autoimmunerkrankungen, Erkrankungen des Verdauungstrakts, Arteriosklerose, Schlaganfall, Infarkt, krankhafte Veränderungen des Blutgefäßsystems und des Herzens, des Lymphsystems, der Bauchspeicheldrüse, der Leber, der Niere, des Gehirns und der Blase, sowie Erkrankungen der elektrischen Reizweiterleitung und neurodegenerative Erkrankungen. Insbesondere ist die Verwendung der Partikel als MRI Kontrastmittel zur bildgebenden Darstellung von Leber Milz und zur Diagnose von Erkrankungen dieser Organe bevorzugt. Weiterhin ist die Verwendung der Partikel als MRI Kontrastmittel zur Angiographie.

Die nachfolgenden Beispiele erläutern die Erfindung ohne Sie auf die konkreten Ausgestaltungen zu beschränken. Der Fachmann ist dazu in der Lage eine Vielzahl von Variationen der in den Experimenten genannten Mengen und Temperaturen aufzufinden, die alle im Umfang der Erfindung liegen, der durch die angefügten Ansprüche bestimmt wird.

### Beschreibung der Figuren und Abbildungen

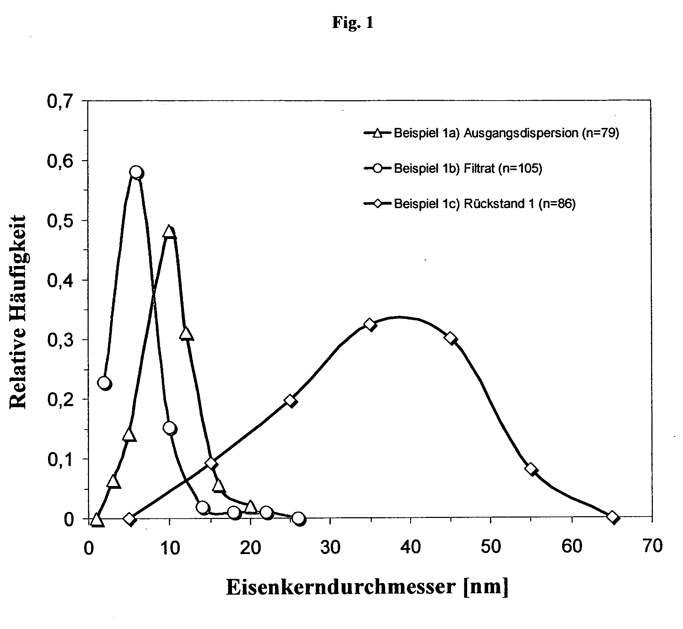

- **Fig. 1**: Stellt die Häufigkeitsverteilung der Größe der Kerne der hergestellten Teilchen dar, wobei die Häufigkeit zwischen 0 und 1 variiert. (entspricht 0% bis 100%).

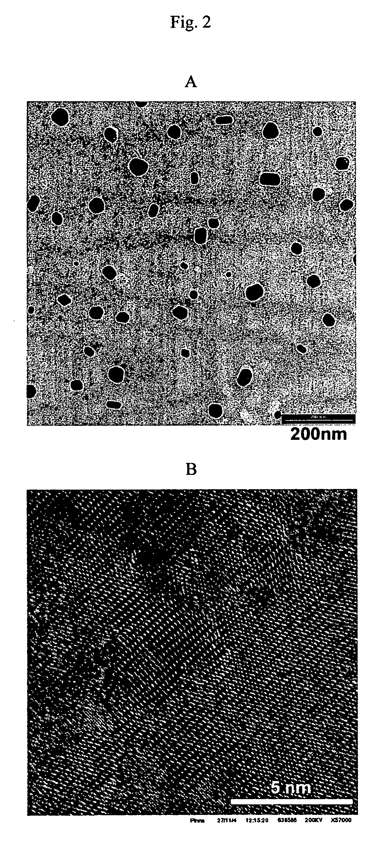

- **Fig. 2**: Die mit A gekennzeichnete Abbildung zeigt eine TEM Aufnahme mit einer Zusammensetzung gemäß Beispiel 1c) (Rückstand 1). Es sind einige nichtkugelförmige Kerne mit einem mittleren Durchmesser von etwa 35 nm zu erkennen. Die mit B gekennzeichnete Abbildung zeigt eine hochauflösende TEM eines großen Kerns einer Zubereitung gemäß 1c) (Rückstand 1). Man erkennt eine Anhäufung von kleinen Monokristallen, die zu einem größeren Polykristall aggregiert sind.

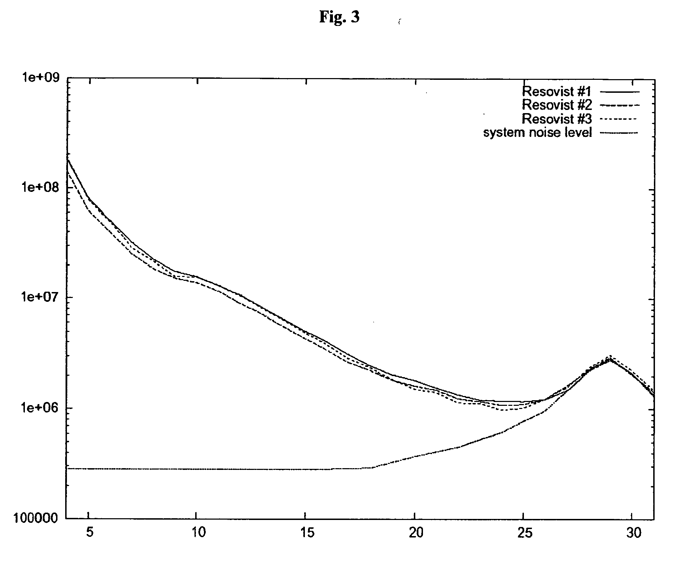

- **Fig. 3**: Ergebnisse einer MPI-Untersuchung mit drei verschiedenen handelsüblichen Resovist^{®-}Chargen. Als Ergebnis aufgetragen ist die Fourieramplitude des gewonnenen Signals (y-Achse) bei der angegebenen Vielfachen der Drive-Feld-Frequenz (x-Achse).

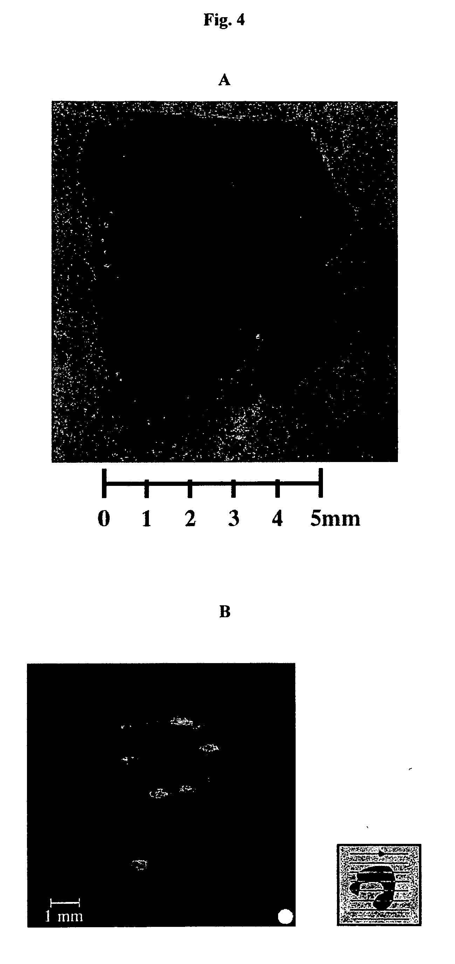

- **Fig. 4**: Die mit A gekennzeichnete Abbildung zeigt ein Phantom zum Erzeugen von Bildern mit einer Anordnung und mit einem Verfahren nach der DE 101 51 778. Dieses Phantom enthält eine Vielzahl von Hohlräumen (im Bild als dunkle Punkte dargestellt), die mit Resovist® gefüllt sind. Abbildung B zeigt ein Bild dieses Phantoms, wobei die mit Resovist® gefüllten Hohlräume als helle Bereiche erscheinen.

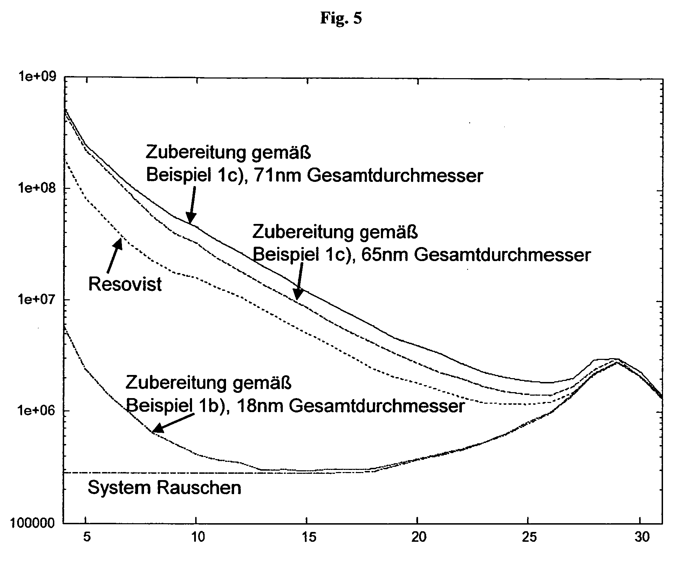

- **Fig. 5**: Das Ergebnis einer MPI-Messung mit der Zubereitung gemäß Beispiel 1b) und 1c) (zwei verschiedene gemittelte Gesamtdurchmesser) ist im Vergleich zu handelsüblichen Resovist® dargestellt. Als Ergebnis aufgetragen ist die Fourieramplitude des gewonnenen Signals (y-Achse) bei der angegebenen Vielfachen der Drive-Feld Frequenz (x-Achse).

### Beispiele

### Beispiel 1: Herstellung der magnetischen Eisenoxidpartikel

### Beispiel 1a) Herstellung der Ausgangspartikeldispersion

105 g Carboxydextran (CDX) mit einer intrinsischen Viskosität von 0.050dl/g wird in 350 ml Wasser gelöst. Unter Stickstoffeinleitung wird dazu eine wäßrige Lösung aus 13.6 g Eisen(II)-chlorid-tetrahydrat und 140 ml 1 M Eisen(III)-chlorid-Lösung (entsprechend 37.8 g Eisen (III)-chlorid-hexahydrat) gegeben. Dazu werden unter Rühren, über 15 min, 242 ml 3 N NaOH-Lösung gegeben und auf 80°C erwärmt. Durch Zugabe von 6 N HCl wird der pH auf 7.0 eingestellt. Es wird 1 h unter Rückfluß weitergerührt.

Nach dem Abkühlen wird die Probe zentrifugiert (2.100 g/ 30 min). Es erfolgt eine Ethanolzugabe entsprechend 78% des Volumens vom Überstand des Präzipitats. Die Probe wird erneut zentrifugiert (2100 g/10 min). Das Präzipitat wird in Wasser gelöst und gegen Wasser dialysiert (16 h). Das Dialysat wird mit NaOH auf pH 7.2 eingestellt und unter reduziertem Druck aufkonzentriert. Das Konzentrat wird durch einen Membranfilter filtriert (Porengröße: 0.2 µm) um 186 mL einer Suspension von Carboxydextran stabilisierten Eisenoxidpartikel zu erhalten. Das Herstellungsverfahren entspricht dem Herstellungsverfahren für Partikel die in Resovist® enthalten sind.

Eisenkonzentration: 52 mg/ml (Eisengehalt: 91%), Partikeldurchmesser der magnetischen Eisenoxide: 9 nm, Gesamtpartikeldurchmesser: 61 nm, Wasser-lösliches Carboxypolysaccharid/Eisen Gewichtsverhältnis 1.15, Magnetisierung bei 1 Tesla: 98 emu/g Eisen, T₂-Relaxivität: 240 (mM sec)⁻¹

### Beispiel 1b) Herstellung kleiner Partikel

Ein magnetischer Filter wird aus einem Ringmagneten (NE 1556, IBS Magnet Berlin, Außendurchmesser 15 mm, Innendurchmesser 5 mm, Höhe 6 mm) und einem im Innenvolumen des Ringmagneten angeordneten Separationsraum aufgebaut. Der Separationsraum besteht aus einer Wandung aus Kunststoff, und wird mit Eisenschrotkugeln (Durchmesser ca. 0,3 mm) gefüllt. Durch den magnetischen Filter werden 0,8 ml einer Dispersion von Eisenoxidpartikeln (gemäß Beispiel 1a) mit einem Eisengehalt von 500 mmol/l und einer T₂-Relaxivity von cirka 240 (mM · sec)⁻¹ mittels hydrostatischem Druck filtriert. Die T₂-Relaxivity des so gewonnenen Filtrats beträgt 41 (mM · sec)⁻¹.

### Beispiel 1c) Herstellung der neuen magnetischen Eisenoxidpartikel

Nach Gewinnung der nach Beispiel 1b) filtrierten Eisenoxidpartikel wird der Rückstand im magnetischen Filter nach Abschalten des Magneten durch Spülen gewonnen. Die erhaltene Partikelsuspension (Rückstand 1) zeigt eine T₂-Relaxivity von 293 (mM · sec)⁻¹ wird gemäß Beispiel 2, 3, 4 und 5 charakterisiert.

Bei einer zweiten Durchführung einer Filtration gemäß Beispiel 1.b), diesmal allerdings mit einem schwächeren Magnetfeld, zeigte der gewonnen Rückstand (Rückstand 2) nach Abschalten des Magnetfeldes eine T₂-Relaxivity von 388 (mM · sec)⁻¹.

### Beispiel 2: Bestimmung der Gesamtpartikelgröße mittels Dynamischer Lichtstreuung.

Mit einem Particlesizer der Firma Malvern (ZetaSizer 3000 Hs a) werden die mittleren Diffusionskoeffizienten (intensitätsgewichtet) der Partikel gemessen und daraus der mittlere hydrodynamischen Durchmesser berechnet. Dieses Verfahren stellt eine Möglichkeit dar einen gemittelten Gesamtpartikeldurchmesser zu bestimmen. Allerdings wird in diesem Fall der Durchmesser des Eisenoxidkerns plus hydratisierter Hülle bestimmt.

Die Ergebnisse sind in der folgenden Tabelle 1 zusammengestellt:

**Tabelle 1**

| | Gemittelter Gesamtpartikeldurchmesser [nm] |
|---|---|
| Beispiel 1a) | 61 |
| Ausgangsdispersion | |
| (Partikel für Resovist^{®}) | |
| Beispiel 1b) | 18 |
| Filtrat | |
| Beispiel 1c) | |
| Rückstand 1 | 65 |
| Rückstand 2 | 71 |

### Beispiel 3: Bestimmung der Kerngroße mittels Elektronenmikroskopie

Mit Hilfe der Transmissionselektronenmikroskopie (TEM) werden die kontrastreichen Eisenoxidkerne, da diese eine höhere Elektronendichte als die Hülle aufweise, vergrößert und photographiert. Die Größe der so abgebildeten Teilchen wird ausgemessen und über den Vergrößerungsfaktor die tatsächliche Kerngröße berechnet. Es werden 50 bis 100 Teilchen ausgezählt und die Ergebnisse in einem Histogramm aufgetragen. Darüber hinaus wird der gemittelte Kerndurchmesser (anzahlgewichtet) berechnet. Die Ergebnisse sind als Histogramm in Fig. 1 und tabellarisch in Tabelle 2 zusammengefaßt.

**Tabelle 2**

| | Gemittelter Kerndurchmesser [nm] |
|---|---|
| Beispiel 1a) | 9 |
| Ausgangsdispersion | |
| (Partikel für Resovist^{®}) | |
| Beispiel 1b) | 6 |
| Filtrat | |
| Beispiel 1c) | |
| Rückstand 1 | 35 |
| Rückstand 2 | nicht bestimmt |

### Beispiel 4: Bestimmung von Gesamtdurchmesser/Kern-Verhältnis.

Aus den Durchmessern gemäß Beispiel 2 und den Kerngrößen gemäß Beispiel 3 wird der Quotient gebildet und in folgender Tabelle 3 zusammengefaßt.

**Tabelle 3**

| | Gemittelter Gesamtpartikeldurchmesser [nm] | Gemittelter Kerndurchmesser [nm] | Gesamtdurchmesser/Kern-Verhältnis |
|---|---|---|---|
| Beispiel 1a) | 61 | 9 | 6.78 |
| Ausgangsdispersion | | | |
| Beispiel 1b) | 18 | 6 | 3.00 |
| Filtrat | | | |
| Beispiel 1c) | | | |
| Rückstand 1 | 65 | 35 | 1.86 |
| Rückstand 2 | 71 | nicht bestimmt | -- |

### Beispiel 5: Identifizierung der Polykristallinität mittels Elektronenmikroskopie und Hochauflösender Elektronenmikroskopie.

Die Polykristallinität der Partikel wurde durch Elektronenmikroskopie und Hochauflösender Elektronenmikroskopie nach Standardverfahren wie z.B. Transmissions Elektronen Mikroskopie (TEM) auf eine CM2000 FEG (Philips) Mikroskop bei 200kV (HRTEM). Die Proben wurden auf einer perforierten Kohlenstoffolie auf einem Kupfernetz abgeschieden. Die Partikel des Rückstands 1 wurden so untersucht. Die Ergebnisse sind mit unterschiedlicher Auflösung in der Figur 2A und 2B dargestellt.

### Beispiele 6: MPI Bildgebung mit magnetischen Eisenoxidpartikeln

### Beispiel 6a) Probenbereitung für MPI Experimente:

In eine PVC-Platte (Dicke 1 mm, laterale Ausdehnung etwa 2 x 2 mm²) wird ein Loch mit einem Durchmesser von 0,5 mm gebohrt. Eine Seite wird mit Tesa-Film verschlossen. Dann wird mit Hilfe eines dünnen Kupferdrahts (0,2 mm lackiert) tröpfchenweise Substanz in das Loch gefüllt, bis es vollständig gefüllt ist. Die Substanz wurde unverdünnt verwendet. Die offene Seite des Lochs wird mit Tesa-Film verschlossen und das Objekt auf einen GFK-Messstab geklebt. Dabei wird die Probe von allen Seiten mit Acrylat-Kleber versiegelt.

Die Probe wird in einen MPI-Scanner wie in DE 101 51 778 A1 beschrieben eingebaut und vermessen. Im Gegensatz zur Bilderzeugung, wie sie in der DE 101 51 788 A1 beschrieben ist, wird die Probe nur hinsichtlich der gemessenen Signalstärke bei unterschiedlichen Frequenzen untersucht. Dazu wird in dem MPI-Scanner ein Gradientenmagnetfeld verwendet, wie es in Fig. 2 aus der DE 101 51 778 A1 und der dazugehörigen Beschreibung erläutert ist. Die maximale Gradientenfeldstärke beträgt 3.4 T/m/µ0. Diesem Gradientenfeld wird ein magnetisches Wechselfeld überlagert, wie es in Fig. 4a der DE 101 51 778 A1 mit H(t) bezeichnet ist. Die Amplitude des Wechselfeldes beträgt 10 mT/µ0 in Richtung des maximalen Gradienten des Gradientenfeldes, die Frequenz 25.25kHz. Die Probe kann in dem MPI-Scanner mechanisch verfahren werden, so daß Daten von verschiedenen Meßpunkten akquiriert werden können. Vorliegend werden 52x52 Meßpunkten aufgenommen, die auf einer Fläche von 10x10mm² verteilt sind. Die Meßzeit an jedem Punkt beträgt 0.4s.

Von den an den 52x52 Meßpunkten aufgenommen Signalen wird der Signalwert mit der größten und der kleinsten Signalstärke ermittelt. Diese Signalwerte sind Fourier-Transformierte Signale und daher komplexe Werte. Es wird zunächst die Differenz zwischen den Realteilen des größten und kleinsten Signalwertes und die Differenz zwischen den Imaginärteilen des größten und kleinsten Signalwertes gebildet. Als Ergebnis aufgetragen ist die Quadratwurzel aus der Quadtratsumme der beiden ermittelten Differenzen (y-Achse bzw. vertikale Achse) bei der angegebenen Vielfachen der Frequenz des magnetischen Wechselfeldes (x-Achse bzw. horizontale Achse).

### Beispiel 6b) MPI mit Resovist®

Resovist® Marktware wird gemäß Beispiel 6a) vorbereitet und vermessen. Die Eisenkonzentration in Resovist® beträgt 500 mmol Fe/L. Die Ergebnisse sind in der Fig. 3 zusammengefaßt. Es wurde ein Signal/Rausch-Abstand bis etwa zum 25-fachen der Drive-Feld Frequenz beobachtet. Weiterhin wurden in unabhängigen Experimenten reproduzierbare Ergebnisse erhalten (3 verschiedene Resovist® Chargen). Somit konnte nachgewiesen werden dass sich Resovist zur MPI Bildgebung eignet.

### Beispiel 6c) MPI mit Zubereitung gemäß Beispiel 1b)

Die Eisenkonzentration einer Suspensionen gemäß Beispiel 1b) wird auf 500mmoL Fe/L eingestellt und anschließend gemäß Beispiel 6a) vorbereitet und vermessen.

Die Ergebnisse sind in Abbildung unter 5) zusammen mit den Resultaten der Vergleichsprobe und den Ergebnissen aus Beispiel 6d) dargestellt.

Es wurde gefunden, daß die Zusammensetzung nach Beispiel 1b) (Filtrat) ein deutlich schlechteres Signal/Rauschverhältnis als die Zusammensetzung nach Beispiel 1a) (Ausgangsdispersion) bzw. Resovist® zeigt.

### Beispiel 6d) MPI mit Zubereitungen gemäß Beispiel 1c)

Die Eisenkonzentration der Suspensionen gemäß Beispiel 1c) (Rückstand 1 und Rückstand 2) werden auf 500mmoL Fe/L eingestellt und anschließend gemäß Beispiel 6a) vorbereitet und vermessen. Die Ergebnisse sind in der Fig. 5 zusammen mit den Resultaten der Vergleichsprobe und den Ergebnissen aus Beispiel 6c dargestellt.

Es wurde gefunden, daß die Zusammensetzung enthaltend die neuen, erfindungsgemäßen Partikel gemäß Zubereitung nach Beispiel 1c) bessere Signal/Rauschverhältnisse als die Ausgangsdispersion gemäß Beispiel 1a) (bzw. der Vergleichsprobe eines kommerziell erhältlichen MR-Kontrastmittels, Resovist ®), und des Filtrats gemäß Beispiel 1b) zeigen.

Die neuen, erfindungsgemäßen Partikel gemäß Zubereitung nach Beispiel 1c) sind demnach für eine verbesserte MPI-Bildgebung geeignet.

### Beispiel 6e) MPI Bildgebung mit Resovist®

Fig. 4 zeigt in Bild A die Abbildung eines Phantoms zum Erzeugen von Bildern mit einer Anordnung und mit einem Verfahren nach der DE 101 51 778. Dieses Phantom enthält eine Vielzahl von Hohlräumen (im Bild als dunkle Punkte dargestellt), die mit Resovist® gefüllt sind.

Fig. 4 zeigt in Bild B ein Bild dieses Phantoms, wobei die mit Resovist® gefüllten Hohlräume als helle Bereiche erscheinen.

### Beispiel 7: Synthese von Chelator-Eisenoxidpartikel und Kopplung von multi-His-L-Selectin (MECA79) als in vivo Kontrastmittel

Im folgenden wird die Kopplung von NTA (Nitrilotriessigsäure-Derivat; α-N-[Bis-Carboxymethyl-]Lysin) an Carboxydextran stabilisierten magnetischen Eisenoxidpartikeln hergestellt gemäß Beispiel 1) beschrieben.

Zu diesem Zweck werden die Carboxydextran stabilisierten magnetischen Eisenoxidpartikeln in wäßriger Lösung mit einem 31-fachen Teilchenüberschuß an Natriumperiodat (bezogen auf das Carboxydextran) für 30 min unter Rühren im Dunkeln bei Raumtemperatur (RT) oxidiert. Anschließend wird das Natriumperiodat quantitativ über eine Gelfiltration abgetrennt. Die Dextranmagnetite werden in Phosphatpuffer (0,1 M Phosphatpuffer pH 7,0) eluiert. Anschließend wird NTA zu den oxidierten Dextranmagnetiten hinzugegeben und für 2h bei RT unter gelegentlichem Schütteln im Dunkeln inkubiert. Dabei kann NTA im Überschuß an die Dextranmagnetite gekoppelt werden. Anschließend wird 1/10 Volumen des Reduktionsmittels Dimethylboran (150 mM in H₂O) hinzugegeben und für weitere 2 h bei RT unter gelegentlichem Schütteln im Dunkeln inkubiert. Der letzte Schritt wird wiederholt, gefolgt von einer Inkubation bei 4°C über Nacht. Die Abtrennung des ungebundenen NTA vom an die Oberfläche der Partikel gebundenen NTA erfolgt über Gel- oder Ultrafiltration. Die Partikel werden in PBS oder in 0,1 M HEPES (jeweils pH 7,0 - 7,4) eluiert und durch Zugabe von 5 mg/ml Carboxydextran (Endkonzentration) stabilisiert. Die Partikel werden sterilfiltriert, und es wird Natriumazid in einer Endkonzentration von 0,1% hinzugegeben. Anschließend wird der Eisengehalt der Suspension sowie die mittlere Teilchengröße der Partikel bestimmt.

Um die Kopplungseffizienz des NTA an die Partikeloberfläche zu überprüfen, werden die Partikel zunächst mit 10 mM EDTA in PBS oder 0,1 M HEPES für 1 h bei RT unter gelegentlichem Schütteln inkubiert. Anschließend wird das EDTA über Gel- oder Ultrafiltration abgetrennt, und die Probe mit Co²⁺, Ni²⁺ oder vergleichbaren zweiwertigen Ionen inkubiert, die von dem Chelator komplexiert werden.

Überschüssige Ionen werden dann über Gel- oder Ultrafiltration von den Partikeln abgetrennt. Die Anzahl an auf der Partikeloberfläche gebundenen NTA-Molekülen kann durch eine ICP-Messung der gebundenen Ionen bei Subtraktion der Ionen, die an unmodifizierte Dextranmagnetite binden, ermittelt werden.

### Beispiel 8: Kopplung von multi-His-L-Selectin an NTA-Dextranmagnetite

Die NTA-tragenden Dextranmagnetite werden zunächst mit Ni²⁺ -Ionen (o.ä. Ionen) und dann mit multi-His-getaggten Selectinmolekülen in PBS oder 0,1 M HEPES mit 0,2% Milch (zur Reduktion unspezifischer Bindungen) für 10 min bei RT inkubiert. Ungebundene Selectinmoleküle werden über geeignete Ultrafiltrationseinheiten oder über Magnetsäulen (Miltenyi Biotec) bei angelegtem Magnetfeld abgetrennt. Die resultierenden Kontrastmittelkonstrukte werden in vitro auf ihre Bindungsfähigkeit z.B. im Gefrierschnitt peripherer Maus-Lympknoten überprüft und können dann für *in vivo* Experimente zur Bildgebung eingesetzt.

### Beispiel 9: Kopplung von Streptavidin an magnetischen Eisenoxidpartikel gemäß Beispiel 1).

Die Suspensionen gemäß Beispiel 1) werden durch mindestens 3-malige Sedimentation in der Ultrazentrifuge und gleichvolumige Aufnahme mit 0,02% TritonX100 Natriumacetatpufferlösung (pH 4,5) gereinigt. 1 ml der aufgereinigten Suspension wird mit 1 ml einer 2 %igen Streptavidin-Lösung versetzt und 60 Minuten bei 4 C gerührt. Anschließend werden 10 mg EDC hinzugegeben. Dabei wird der pH Wert kontrolliert. Sollte der pH-Wert von 4.5 +/- 0.2 abweichen, so muß mit 0,01N HCl oder 0,01N NaOH nachgestellt werden.

Die Inkubation wird ca. 16 Stunden bei 4°C unter Rühren fortgeführt und anschließend durch eine 15-minütige Inkubation mit 1 M Ethanolamin abgeschlossen. Die magnetischen Eisenoxidpartikel, an denen Streptavidin gebunden wurde, werden durch mehrmalige Zentrifugation vom ungebundenen Protein und den Nebenprodukten abgetrennt.

Der Kopplungserfolg wird mittels Aggregations-Assay durch Zugabe von mehrfach Biotinmodifizierten BSA nachgewiesen. Nach Zugabe von Biotin-BSA aggregiertem Streptavidin führen funktionalisierte Eisenoxidpartikel zu sichtbaren Aggregaten, dagegen zeigen unbehandelte Eisenoxidpartikel keinerlei Aggregation und bleiben stabil in Dispersion.

Eine quantitative Aussage zum Kopplungserfolg kann mittels Surface Plasmon Resonance (BioCore, BioCore2000) an immobilisiertem Biotin-BSA erhalten werden.

### Beispiel 10: Bindung von MECA79-Antikörper an Streptavidin funktionalisierte magnetische Eisenoxidpartikel gemäß Beispiel 9).

Die magnetischen Eisenoxidpartikel, an denen gemäß Beispiel 9) Streptavidin gebunden wurde, werden durch zweimalige Zentrifugation gegen Hepes-Puffer / TritonX100 Lösung 0.01% gereinigt, umgepuffert und aufkonzentriert. Die gereinigten, nun Biotin-bindenden Mikrokapseln werden für 1 Stunde mit 1 mg biotinyliertem MECA79-Antikörper inkubiert und anschließend gewaschen. Analog können Kontrollpartikel unter Verwendung eines biotinylierten Isotyp-IgM-Antikörpers (z.B. Klon R4-22) hergestellt werden.

50 % der eingesetzten Antikörpermengen werden an die Mikrokapseln gebunden (BioCore-Messung: Sättigungsreihe mit anti-IgM-FITC Antikörpern). Der MECA79-Antikörper erkennt das "periphere node Adressin", eine Ligandengruppe, die konstitutiv präsentiert nur auf den hochendothelialen Venolen der peripheren und mesenterialen Lymphknoten vorkommen.

### Beispiel 11: Kopplung von Anti-Maus-CD105-Antikörper an magnetische Eisenoxidpartikel

Biotinylierte Anti-Maus-CD105-Antikörper werden analog Beispiel 10) an Streptavidin funktionalisierte magnetische Eisenoxidpartikel gemäß Beispiel 9) gebunden. Der CD105-Antikörper erkennt Angiogenese-spezifische Rezeptoren und kann zur bildgestützten Diagnose von Tumoren verwendet werden

### Beispiel 12: Kopplung von Anti-Maus-ICAM-1-Antikörper an magnetische Eisenoxidpartikel

Biotinylierte Anti-Maus-ICAM-1-Antikörper werden analog Beispiel 10 an Streptavidin funktionalisierte magnetische Eisenoxidpartikel gemäß Beispiel 9) gebunden. Der ICAM-1-Antikörper erkennt Entzündungsherde beispielsweise im experimentellen autoimmunen Encephalomyelitismodell (EAE) der Maus. Das EAE-Modell gilt als *in vivo* Krankheitsmodell für Multiple Sklerose.

## Patentansprüche

1. Partikel umfassend in einer pharmazeutisch annehmbaren Hülle einen magnetischen Eisenoxidkern mit einem Durchmesser von 20 nm bis 1 µm, **dadurch gekennzeichnet, daß** das Gesamtpartikeldurchmesser-Kerndurchmesserverhältnis kleiner als 6 ist.

2. Partikel nach Anspruch 1, **dadurch gekennzeichnet, daß** die pharmazeutisch annehmbare Hülle die kolloidale Lösung stabilisiert.

3. Partikel nach Anspruch 1 oder 2, **dadurch gekennzeichnet, daß** die pharmazeutisch annehmbare Hülle ein synthetisches Polymer oder Copolymer, eine Stärke oder ein Derivat davon, ein Dextran oder ein Derivat davon, ein Cyclodextran oder Derivat davon, eine Fettsäure, ein Polysaccharid, ein Lecithin oder ein Mono-, Di- oder Triglycerid oder ein Derivat davon oder Gemische davon umfaßt.

4. Partikel nach Anspruch 3, wobei
(i) das synthetische Polymer oder Copolymer ausgewählt ist aus der Gruppe bestehend aus Polyoxyethylensorbitanestern, Polyoxyethylen und Derivaten davon, Polyoxypropylen und Derivaten davon, nichtionischen Oberflächen-aktiven Substanzen, Polyoxylstearaten (35-80), Polyvinylalkoholen, polymerisierte Sucrose, Polyhydroxyalkylmethacrylamiden, Milchsäure und Glycolsäure-Copolymere, Polyorthoester, Polyalkylcyanoacrylates, Polyethylenglycol, Polypropylenglycol, Polyglycerole, polyhydroxylierte Polyvinylmatrices, Polyhydroxaethylaspartamide, Polyaminosäuren, Styrol- und Maleinsäure-Copolymere, Polycaprolactonen, Carboxypolysaccharid, und Polyanhydriden;
(ii) das Stärkederivate ausgewählt ist aus der Gruppe bestehend aus Stärke-2-hydroxymethylether und Hydroxyethylstärke;
(iii) das Dextran oder Derivat davon ausgewählt ist aus der Gruppe bestehend aus galactosyliertes Dextran, lactosyliertes Dextran, aminiertes Dextran, Dextran mit SH-Gruppen, Dextran mit Carboxylgruppen, Dextran mit Aldehydgruppen, biotinyliertes Dextran
(iv) das Cyclodextrin ausgewählt ist aus der Gruppe bestehend aus beta-Cyclodextrin und Hydroxypropylcyclodextrin
(v) die Fettsäure ausgewählt ist aus der Gruppe bestehend aus Natriumlaurylsulfat, Natriumstearat, Stearinsäure, Sorbitanmonolaurat, Sorbitanmonooleate, Sorbitanmonopalmitat und Sorbitanmonostearat.

5. Partikel nach einem der Ansprüche 1 bis 4, **dadurch gekennzeichnet, daß** das magnetische Eisenoxidkern Magnetit oder Maghemit oder Gemischen davon umfaßt.

6. Partikel nach einem der Ansprüche 1 bis 5, **dadurch gekennzeichnet, daß** der Eisenoxidkern mindestens fünf Eisenoxidmonokristalle enthält.

7. Partikel nach einem der Ansprüche 1 bis 6, **dadurch gekennzeichnet, daß** der Gesamtpartikeldurchmesser 30 nm bis 2 µm beträgt.

8. Partikel nach einem der Ansprüche 1 bis 7, **dadurch gekennzeichnet, daß** der Durchmesser des Eisenoxidkerns 30 bis 500 nm beträgt.

9. Partikel nach einem der Ansprüche 1 bis 8 **dadurch gekennzeichnet, daß** Eisenoxidkern polykristallin ist.

10. Partikel nach einem der Ansprüche 1 bis 9, **dadurch gekennzeichnet, daß** Polyethylenglycol- und/oder Polypropylenglycolreste kovalent oder nicht-kovalent mit der Oberfläche des Partikels verbunden sind.

11. Verfahren zur Herstellung von Partikeln, die in einer pharmazeutisch annehmbaren Hülle einen magnetischen Eisenoxidkern mit einem Durchmesser von 20 nm bis 1 µm umfassen, deren Gesamtpartikeldurchmesser-Kerndurchmesserverhältnis kleiner als 6 ist, umfassend die folgenden Schritte:
(i) Mischen einer wäßrigen Mischeisensalzlösung, die ein Eisen-(II)-Salz und eine Eisen-(III)-Salz enthält, mit einer wäßrigen Alkalilösung in Gegenwart eines synthetischen Polymers oder Copolymers, einer Stärke oder eines Derivats davon, eines Dextrans oder ein Derivats davon, eines Cyclodextrans oder Derivats davon, einer Fettsäure, eines Polysaccharid, eines Lecithins oder eines Mono-, Di- oder Triglycerids oder eines Derivat davon oder Gemischen davon, bis zur Bildung einer kolloidalen Lösung der Partikel,
(ii) Durchleiten der Partikel-haltigen Lösung durch ein magnetisches Gradientenfeld,
(iii) Entfernung des magnetischen Gradientenfelds,
(iv) und Gewinnung der im Gradientenfeld zurückgehaltenen Partikel.

12. Verfahren nach Anspruch 11, **dadurch gekennzeichnet, daß** in einem weiteren Schritt Partikel eines vorbestimmten Gesamtpartikeldurchmessers ausgewählt werden.

13. Partikel herstellbar mit einem Verfahren nach Anspruch 11 oder 12.

14. Zusammensetzung, **dadurch gekennzeichnet, daß** mindestens 2% der in der Zusammensetzung enthaltenden Partikel, die in einer pharmazeutisch annehmbaren Hülle einen magnetischen Eisenoxidkern enthalten, Partikel gemäß einem der Ansprüche 1 bis 10 oder ein Partikel nach Anspruch 13 sind.

15. Zusammensetzung nach Anspruch 14, **dadurch gekennzeichnet, daß** der Partikeldurchmesser gemäß einem der Ansprüche 1 bis 10 oder der Partikel nach Anspruch 13 für mindestens 50% der Partikel in einem Bereich von 10% um den gemittelten Partikeldurchmesser der Partikel liegt.

16. Flüssigkeit **dadurch gekennzeichnet, daß** sie eine Zusammensetzung nach Anspruch 14 oder 15 enthält.

17. Flüssigkeit nach Anspruch 16, **dadurch gekennzeichnet, daß** die Flüssigkeit in Form einer stabilisierten kolloidalen Lösung vorliegt.

18. Verwendung einer Zusammensetzung nach Anspruch 14 oder 15 oder einer Flüssigkeit nach Anspruch 16 oder 17 zur Herstellung eines Diagnostikums für die Verwendung bei Bildgebung durch Detektion magnetischer Partikel (MPI) zur Diagnose einer Erkrankung ausgewählt aus der Gruppe bestehend aus proliferativen Erkrankungen, inflammatorischen Erkrankungen, Autoimmunerkrankungen, Erkrankungen des Verdauungstrakts, Arteriosklerose, Schlaganfall, Infarkt, krankhafte Veränderungen des Blutgefäßsystems, des Lymphsystems, der Bauchspeicheldrüse, der Leber, der Niere, des Gehirns und der Blase, sowie Erkrankungen der elektrischen Reizweiterleitung und neurodegenerative Erkrankungen.

19. Verwendung einer Zusammensetzung **dadurch gekennzeichnet, daß** mindestens 2% der in der Zusammensetzung enthaltenden Partikel in einer pharmazeutisch annehmbaren Hülle einen magnetischen Eisenoxidkern mit einem Durchmesser von 20 nm bis 1 µm umfassen, deren Gesamtpartikeldurchmesser-Kerndurchmesserverhältnis kleiner als 6 ist, zur Herstellung eines Diagnostikums für die Verwendung bei der Bildgebung durch Detektion magnetischer Partikel (MPI) zur Diagnose einer Erkrankung ausgewählt aus der Gruppe bestehend aus proliferativen Erkrankungen, inflammatorischen Erkrankungen, Autoimmunerkrankungen, Erkrankungen des Verdauungstrakts, Arteriosklerose, Schlaganfall, Infarkt, krankhafte Veränderungen des Blutgefäßsystems, des Lymphsystems, der Bauchspeicheldrüse, der Leber, der Niere, des Gehirns und der Blase, sowie Erkrankungen der elektrischen Reizweiterleitung und neurodegenerative Erkrankungen.

20. Verwendung nach Anspruch 18 oder 19, wobei die Detektion der Partikel durch ein Verfahren umfassend die folgenden Schritte erfolgt:
(i)) Erzeugung eines Magnetfeldes mit einem solchen räumlichen Verlauf der magnetischen Feldstärke, daß sich in dem Untersuchungsbereich ein erster Teilbereich mit niedriger magnetischer Feldstärke und ein zweiter Teilbereich mit höherer magnetischer Feldstärke ergibt,
(ii) Veränderung der räumlichen Lage der beiden Teilbereiche in dem Untersuchungsbereich, so daß sich die Magnetisierung der Partikel örtlich ändert,
(iii) Erfassung von Signalen, die von der durch diese Veränderung beeinflußten Magnetisierung im Untersuchungsbereich abhängen,
(iv) Auswertung der Signale zur Gewinnung von Information über die räumliche Verteilung der magnetischen Partikel im Untersuchungsbereich.

21. Verwendung nach Anspruch 20, **dadurch gekennzeichnet, daß** die Anordnung zur Detektion die folgenden Mittel umfaßt:
a) Mittel zum Erzeugen eines Magnetfeldes mit einem solchen räumlichen Verlauf der magnetischen Feldstärke, daß sich in dem Untersuchungsbereich ein erster Teilbereich mit niedriger magnetischer Feldstärke und ein zweiter Teilbereich mit höherer magnetischer Feldstärke ergibt,
b) Mittel zum Verändern der räumlichen Lage der beiden Teilbereiche in dem Untersuchungsbereich, so daß die Magnetisierung der Partikel sich örtlich ändert
c) Mittel zur Erfassung von Signalen, die von der durch die Veränderung der räumlichen Lage beeinflußten Magnetisierung im Untersuchungsbereich abhängen,
d) Mittel zur Auswertung der Signale zur Gewinnung von Information über die räumliche Verteilung der magnetischen Partikel im Untersuchungsbereich.

22. Verwendung nach einem der Ansprüche 18 bis 21, **dadurch gekennzeichnet, daß** die proliferative Erkrankung ausgewählt ist aus der Gruppe bestehend aus einem Tumor, einer Präcancerose, eine Dysplasie, einer Endometriose und einer Metaplasie.

23. Verwendung nach einem der Ansprüche 18 bis 21, **dadurch gekennzeichnet, daß** die Autoimmunerkrankung ausgewählt ist aus der Gruppe bestehend aus rheumatoider Arthritis, inflammatorischer Darmerkrankung, Osteoarthritis, neuropathischen Schmerzen Alopecia areta, Psoriasis, psoriatische Arthritis, akute Pancreatitis, Allograftabstoßung, Allergien, allergische Entzündungen der Lunge, Multiple Sclerosis, Alzheimer, Morbus Crohn, und systemischer Lupus erythematosus.
